# EUROPÄISCHE PATENTANMELDUNG

(11) **EP 2 014 274 A1**
(43) Veröffentlichungstag der Anmeldung: **14.01.2009**
(21) Anmeldenummer: 07011967.2
(22) Anmeldetag: 19.06.2007
(51) Int. Cl.: A61K 8/31, A61K 31/01, A61Q 5/00, A61Q 17/00, A61Q 19/00

(54) **Kohlenwasserstoff Gemische und ihre Verwendung**

(71) Anmelder: Cognis IP Management GmbH, 40589 Düsseldorf (DE)
(72) Erfinder: Dierker, Markus, 40597 Düsseldorf (DE); Ansmann, Achim, 40699 Erkrath (DE)

(57) **Zusammenfassung**

Die vorliegende Erfindung betrifft Kohlenwasserstoff Gemische enthaltend lineare C11 und lineare C13 Kohlenwasserstoffe, wobei die Summe der linearen C11- und linearen C13-Kohlenwasserstoffe größer gleich 60 Gew. % bezogen auf die Summe der Kohlenwasserstoffe, beträgt, sowie die Verwendung dieser Gemische in kosmetischen und/oder pharmazeutischen Zubereitungen.

## Beschreibung

Die vorliegenden Erfindung betrifft Kohlenwasserstoff Gemische, ihre Verwendung in kosmetischen und/oder pharmazeutischen Zubereitungen, sowie kosmetische und/oder pharmazeutische Zubereitungen, enthaltend Kohlenwasserstoff Gemische.

### Stand der Technik

Sensorisch leichte Ölkörper, so genannte "light emollients", werden von der kosmetischen Industrie in einer Vielzahl von Formulierungen verwendet. Insbesondere für die dekorative Kosmetik bzw. in pflegenden Formulierungen werden so genannte "leichte" Komponenten eingesetzt. Bei diesen Komponenten kann es sich beispielsweise um flüchtige, cyclische Silikone (z.B. Cyclopentasiloxan oder Cyclomethicone) oder Kohlenwasserstoffe aus petrochemischen Prozessen handeln. Bei den zuletzt genannten Stoffen handelt es sich aufgrund ihrer Herstellung überwiegend um Gemische aus linearen, cyclischen und verzweigten Kohlenwasserstoffen, deren Flammpunkt durchaus unter 50 °C (wie z.B. beim Isododecan) liegen kann. Beispiele und anwendungstechnische Beschreibungen derartiger Formulierungen können in Standardwerken, wie zum Beispiel: **,**Handbook of Cosmetic Science and Technology', A. Barel, M. Paye, H. Maibach, Marcel Dekker Inc. 2001 nachgelesen werden. Aus toxikologischen, ökologischen bzw. sicherheitstechnischen Gründen besteht jedoch in Zukunft Bedarf nach alternativen Rohstoffen für derartige Formulierungsaufgaben.

In kosmetischen und pharmazeutischen Zubereitungen werden unter der Bezeichnung "Mineral Öl" die aus mineralischen Rohstoffen (Erdöl, Braun- u. Steinkohlen) gewonnenen flüssigen Destillations-Produkte, die im Wesentlichen aus Gemischen von gesättigten Kohlenwasserstoffen mit linearer, cyclischer und/oder verzweigter Struktur bestehen, eingesetzt. Diese Kohlenwasserstoff Gemische müssen jedoch aufwendig gereinigt und chemisch modifiziert werden, bevor sie den Anforderungen an kosmetische Rohstoffe entsprechen.

Die Aufgabe der Erfindung bestand darin, alternative Rohstoffe zu finden, die ökologisch bzw. toxikologisch unbedenklich sind. Dabei war es insbesondere von Interesse Rohstoffe bereit zu stellen, welche ohne aufwendige Reinigungsschritte direkt in kosmetischen bzw. pharmazeutischen Zubereitungen eingesetzt werden können. Vorzugsweise sollten diese Rohstoffe auf Basis nachwachsender Rohstoffe erhältlich sein. Diese Rohstoffe sollten in typischen kosmetischen und/oder pharmazeutischen Formulierungen ohne anwendungstechnische Einschränkungen direkt eingesetzt werden können. Darüber hinaus sollten die Rohstoffe gegenüber den Kohlenwasserstoff Gemischen des Standes der Technik eine verbesserte Sensorik aufweisen, wünschenswert war auch, dass diese Rohstoffe eine bessere Hautverträglichheit aufweisen.
Eine weitere Aufgabe bestand darin Rohstoffe zur Verfügung zu stellen, welche eine stabile Formulierung mit AP/Deo (=Antiperspirant/Desodorant) Wirkstoffen ermöglicht. Kosmetische Zubereitungen der Kategorie Antiperspirantien/Desodorantien, insbesondere in so genannten "Stick-Formulierungen" haben immer noch das Problem der unzureichenden Stabilität der kosmetischen Grundlage, insoweit daß sich geruchliche Veränderungen während der Lagerung ergeben. Eine weitere Aufgabe der Erfindung bestand daher darin, Rohstoffe zu Verfügung zu stellen, welche es ermöglichen antiperspirierende bzw. desodorierende Zubereitungen, insbesondere solche in "Stick-Formulierung" stabil bereit zu stellen. Diese Zubereitungen sollten insbesondere bei längerer Lagerung keine unerwünschten Geruchsentwicklungen zeigen. Eine weitere Aufgabe bestand darin Rohstoffe zu Verfügung zu stellen, welche einen sensorisch "leichten" Eindruck vermitteln, insbesondere in Kombination mit UV-Lichtschutzfiltern.

### Beschreibung der Erfindung

Ein Gegenstand der Erfindung betrifft Kohlenwasserstoff Gemische enthaltend lineare C11 und lineare C13 Kohlenwasserstoffe, wobei die Summe der linearen C11- und linearen C13-Kohlenwasserstoffe größer gleich 60 Gew. % bezogen auf die Summe der Kohlenwasserstoffe, beträgt.

Die Bezugsgröße "Summe der Kohlenwasserstoffe" umfasst alle im Gemisch enthaltenen Kohlenwasserstoffe, unabhängig von ihrer Kohlenstoffzahl.

Als Kohlenwasserstoffe werden organische Verbindungen bezeichnet, die nur aus Kohlenstoff und Wasserstoff bestehen. Sie umfassen sowohl cyclische als auch acyclische (=aliphatische) Verbindungen. Sie umfassen sowohl gesättigte wie einfach oder mehrfach ungesättigte Verbindungen. Die Kohlenwasserstoffe können linear oder verzweigt sein. Je nach Anzahl der Kohlenstoffatome im Kohlenwasserstoff kann man die Kohlenwasserstoffe einteilen in ungradzahlige Kohlenwasserstoffe (wie beispielsweise Nonan, Undecan, Tridecan) oder geradzahlige Kohlenwasserstoffe (wie beispielsweise Octan, Dodecan, Tetradecan). Je nach Art der Verweigung kann man die Kohlenwasserstoffe einteilen in lineare (= unverzweigte) oder verzweigte Kohlenwasserstoffe. Gesättigte, aliphatische Kohlenwasserstoffe werden auch als Paraffine bezeichnet.

Als "Kohlenwasserstoff Gemisch" im Sinne der Erfindung werden Mischungen von Kohlenwasserstoffen verstanden, die bis zu 10 Gew.-% Substanzen enthalten, die nicht zu den Kohlenwasserstoffen zählen. Die Gew.-% Angaben der linearen C11 und linearen C13 Kohlenwasserstoffe beziehen sich jeweils auf die Summe der im Gemisch vorhandenen Kohlenwasserstoffe. Die bis zu 10 Gew.-% vorhandenen Nicht-Kohlenwasserstoffe werden für diese Berechnung nicht berücksichtigt.

Bei den Substanzen, die nicht zu den Kohlenwasserstoffen zählen und die bis zu 10 Gew.-%, insbesondere bis zu 8 Gew.-%, vorzugsweise bis zu 5 Gew.-% im erfindungsgemäßen Kohlenwasserstoff Gemisch enthalten sein können, handelt sich beispielsweise um Fettalkohole, die als nicht umgesetzte Edukte im Kohlenwasserstoff Gemisch verbleiben.

Der Begriff "CX-Kohlenwasserstoff" umfasst Kohlenwasserstoffe mit einer C-Zahl von X, so umfasst beispielsweise der Begriff C11-Kohlenwasserstoff alle Kohlenwasserstoffe mit einer C-Zahl von 11.

Eine bevorzugte Ausführungsform der Erfindung betrifft Kohlenwasserstoff Gemische, wobei das Gemisch enthält
(a) 50 bis 90 Gew.-% lineare C-11 Kohlenwasserstoffe, vorzugsweise n-Undecan
(b) 10 bis 50 Gew.-% lineare C13 Kohlenwasserstoffe, vorzugsweise n-Tridecan bezogen auf die Summe der Kohlenwasserstoffe.

Besonders bevorzugt sind Kohlenwasserstoff Gemische, welche enthalten
(a) 55 bis 80 Gew.-%, insbesondere 60 bis 75 Gew.-%, insbesondere 65 bis 70 Gew.% lineare C-11 Kohlenwasserstoffe, vorzugsweise n-Undecan
(b) 20 bis 45 Gew.-% insbesondere 24 bis 40 Gew.-%, insbesondere 24 bis 30 Gew.% lineare C-13 Kohlenwasserstoffe, vorzugsweise, vorzugsweise n-Tridecan
bezogen auf die Summe der Kohlenwasserstoffe.

Eine bevorzugte Ausführungsform der Erfindung betrifft Kohlenwasserstoff Gemische, dadurch gekennzeichnet, dass die Summe der linearen C11- und linearen C13-Kohlenwasserstoffe größer gleich 70 Gew.-%, insbesondere größer gleich 80 Gew.-%, bevorzugt größer gleich 90 Gew.%, besonders bevorzugt größer gleich 95 Gew.-%, insbesondere größer gleich 99 Gew.-%, bezogen auf die Summe der Kohlenwasserstoffe, beträgt.

In einer bevorzugten Ausführungsform der Erfindung beträgt das Gewichtsverhältnis von linearen C11-Kohlenwasserstoffen zu linearen C13-Kohlenwasserstoffen 1,5 bis 3,5.

Eine bevorzugte Ausführungsform der Erfindung betrifft Kohlenwasserstoff Gemische enthaltend lineare C11 und lineare C13 Kohlenwasserstoffe, wobei die Summe der linearen C11- und linearen C13-Kohlenwasserstoffe größer gleich 60 Gew. % bezogen auf die Summe der Kohlenwasserstoffe beträgt und wobei die Summe der **verzweigten** Kohlenwasserstoffe kleiner gleich 10 Gew.-%, bezogen auf die Summe der Kohlenwasserstoffe, beträgt.

Besonders bevorzugt sind Kohlenwasserstoff Gemische, enthaltend lineare C11 und lineare C13 Kohlenwasserstoffe, wobei die Summe der linearen C11- und linearen C13-Kohlenwasserstoffe größer gleich 60 Gew. % bezogen auf die Summe der Kohlenwasserstoffe beträgt und wobei die Summe der **verzweigten** Kohlenwasserstoffe kleiner gleich 8 Gew.-%, insbesondere kleiner gleich 5 Gew.-%, insbesondere kleiner gleich 3 Gew.-%, bevorzugt kleiner gleich 2 Gew.-% bezogen auf die Summe der Kohlenwasserstoffe, beträgt. In einer bevorzugten Ausführungsform der Erfindung beträgt die Summe der verzweigten Kohlenwasserstoffe kleiner gleich 1 Gew.-% bezogen auf die Summe der Kohlenwasserstoffe.

Besonders bevorzugt sind Kohlenwasserstoff Gemische, bei denen die linearen C11 und/oder linearen C13 Kohlenwasserstoffe **gesättigten** Kohlenwasserstoffe sind, vorzugsweise ist sowohl der lineare C11 als auch der lineare C13 Kohlenwasserstoff ein linearer Kohlenwasserstoff (n-Undecan und n-Tridecan).

In einer bevorzugten Ausführungsform enthalten die erfindungsgemäßen Kohlenwasserstoff Gemische kleiner gleich 10 Gew-%, insbesondere kleiner gleich 5 Gew.-%, bevorzugt kleiner gleich 3 Gew.-%, an **C-12 Kohlenwasserstoffen** bezogen auf die Summe der Kohlenwasserstoffe.

Eine bevorzugte Ausführungsform der Erfindung betrifft Kohlenwasserstoff Gemische enthaltend lineare C11 und lineare C13 Kohlenwasserstoffe, wobei die Summe der linearen C11- und linearen C13-Kohlenwasserstoffe größer gleich 60 Gew. % bezogen auf die Summe der Kohlenwasserstoffe beträgt und wobei die **Summe der Kohlenwasserstoffe mit einer C-Kettenlänge größer gleich 14,** kleiner gleich 15 Gew.-%, bezogen auf die Summe der Kohlenwasserstoffe, beträgt. Besonders bevorzugt sind Kohlenwasserstoff Gemische, bei denen die Summe der Kohlenwasserstoffe mit einer C-Kettenlänge kleiner gleich 14, kleiner gleich 10 Gew.-%, insbesondere kleiner gleich 8 Gew.-%, bevorzugt kleiner gleich 4 Gew.-%, insbesondere kleiner gleich 2 Gew.-% beträgt, jeweils bezogen auf die Summe der Kohlenwasserstoffe.

Eine bevorzugte Ausführungsform der Erfindung betrifft Kohlenwasserstoff Gemische, enthaltend lineare C11 und lineare C13 Kohlenwasserstoffe, wobei die Summe der linearen C11- und linearen C13-Kohlenwasserstoffe größer gleich 60 Gew. % bezogen auf die Summe der Kohlenwasserstoffe beträgt und die Summe der **Kohlenwasserstoffe mit einer C-Kettenlänge von kleiner gleich 10,** kleiner gleich 3 Gew.%, insbesondere kleiner gleich 2 Gew.-%, bevorzugt kleiner gleich 1,5 Gew.-%, insbesondere kleiner gleich 1 Gew.-%, bezogen auf die Summe der Kohlenwasserstoffe, beträgt.

In einer Ausführungsform der Erfindung enthalten die erfindungsgemäßen Kohlenwasserstoff Gemische C12 und C14 Kohlenwasserstoffe und zwar im selben Gewichtsverhältnis zueinander wie die linearen C11 Kohlenwasserstoffe zu den linearen C13 Kohlenwasserstoffe. In einer bevorzugten Ausführungsform der Erfindung beträgt sowohl das Gewichtsverhältnis von linearen C11-Kohlenwasserstoffen zu linearen C13-Kohlenwasserstoffen als auch das Gewichtsverhältnis von C12 zu C14 Kohlenwasserstoffen 1,5 bis 3,5.

In einer Ausführungsform der Erfindung bestehen die Kohlenwasserstoffe im erfindungsgemäßen Kohlenwasserstoff Gemisch aus Kohlenwasserstoffen mit 7 bis 21 Kohlenwasserstoffen, vorzugsweise 9 bis 17 Kohlenwasserstoffen, insbesondere bevorzugt 11 bis 17 Kohlenwasserstoffe.

Die erfindungsgemäßen Kohlenwasserstoff Gemische eignen sich insbesondere zur Verwendung in kosmetischen und/oder pharmazeutischen Zubereitungen, insbesondere als Ölkörper.

### Herstellung der Kohlenwasserstoff Gemische

Die erfindungsgemäßen Kohlenwasserstoff Gemische können beispielsweise durch Abmischen von linearen C11 und linearen C13 Kohlenwasserstoffe erhalten werden. Des Weiteren können sie beispielsweise durch reduktive Demethylierung nach dem Fachmann bekannten Methoden erhalten werden. Besonders geeignet zur Herstellung der erfindungsgemäßen Kohlenwasserstoff Gemische ist das in der internationalen Anmeldung PCT/EP2006/011647 (Cognis) beschrieben Verfahren der reduktiven Dehydroxymethylierung ausgehend von Fettalkoholen pflanzlichen Ursprungs. Dabei können beispielsweise C12 und C14 Fettalkohole einzelnen dem beschriebenen Verfahren unterzogen werden und die so erhaltenen C11 und C13 Kohlenwasserstoffe zu den erfindungsgemäßes Kohlenwasserstoff Gemisch gemischt werden. Bevorzugt ist es jedoch ein Gemisch aus C12 und C14 Fettalkoholen, welches mindestens 60 Gew,-% C12 und C14 Fettalkohole enthält, der reduktiven Dehydroxymethylierung zu unterwerfen, so dass als Reaktionsprodukt direkt das erfindungsgemäßen Kohlenwasserstoff Gemisch erhalten wird. Dieses kann dann direkt, ohne weitere Aufreinigung, in kosmetischen und/oder pharmazeutischen Zubereitung eingesetzt werden.

### Kosmetische und/oder pharmazeutische Zubereitungen

Ein weiterer Gegenstand der Erfindung betrifft **kosmetische und/oder pharmazeutische Zubereitungen,** enthaltend **1 bis 80 Gew.% Kohlenwasserstoffe,** wobei die Summe der linearen C11-und linearen C13-Kohlenwasserstoffe größer gleich 60 Gew. % bezogen auf die Summe der Kohlenwasserstoffe beträgt.

Die Bezugsgröße "Summe der Kohlenwasserstoffe" umfasst alle in der kosmetischen und/oder pharmazeutischen Zubereitung enthaltenen Kohlenwasserstoffe, unabhängig von ihrer Kohlenstoffzahl.

Diese kosmetischen und/oder pharmazeutischen Zusammensetzungen können demnach weitere Kohlenwasserstoffe, wie beispielsweise Paraffine oder Wachse enthalten, solange die Summe der linearen C11- und linearen C13-Kohlenwasserstoffe größer gleich 60 Gew, % bezogen auf die Summe der Kohlenwasserstoffe beträgt.
Diese Zubereitungen können beispielsweise erhalten werden, indem ein erfindungsgemäßes Kohlenwasserstoff Gemisch eingesetzt wird oder indem definierte Mengen an linearen C11 und linearen C13 Kohlenwasserstoffe eingesetzt werden.

In einer bevorzugten Ausführungsform enthalten die kosmetischen und/oder pharmazeutischen Zubereitungen 10 bis 60, insbesondere 5 bis 50, vorzugsweise 3 bis 25 Gew.-% Kohlenwasserstoffe, wobei die Summe der linearen C11- und linearen C13-Kohlenwasserstoffe größer gleich 60 Gew. % bezogen auf die Summe der Kohlenwasserstoffe beträgt.

Besonders bevorzugt sind kosmetische und/oder pharmazeutische Zubereitungen, enthaltend **1 bis 80 Gew.% Kohlenwasserstoffe,** wobei die Kohlenwasserstoffe
- 50 bis 90 Gew.-% lineare C11 Kohlenwasserstoffe, vorzugsweise n-Undecan
- 10 bis 50 Gew.-% lineare C13 Kohlenwasserstoffe, vorzugsweise n-Tridecan bezogen auf die Summe der Kohlenwasserstoffe enthalten.

Besonders bevorzugt sind kosmetische und/oder pharmazeutische Zubereitungen, enthaltend **1 bis 80 Gew.% Kohlenwasserstoffe,** wobei die Kohlenwasserstoffe
- 55 bis 80 Gew.-%, insbesondere 60 bis 75 Gew.-%, insbesondere 65 bis 70 Gew.% lineare C11 Kohlenwasserstoffe, vorzugsweise n-Undecan
- 20 bis 45 Gew.-% insbesondere 24 bis 40 Gew.-%, insbesondere 24 bis 30 Gew.% lineare C13 Kohlenwasserstoffe, vorzugsweise n-Tridecan,
enthalten, bezogen auf die Summe der Kohlenwasserstoffe.

Besonders bevorzugt sind kosmetische und/oder pharmazeutische Zubereitungen, enthaltend **1 bis 80 Gew.% Kohlenwasserstoffe,** wobei die Summe der linearen C11- und linearen C13-Kohlenwasserstoffe größer gleich 70 Gew.-%, insbesondere größer gleich 80 Gew.-%, bevorzugt größer gleich 90 Gew.%, besonders bevorzugt größer gleich 95 Gew.-%, insbesondere größer gleich 99 Gew.-%, bezogen auf die Summe der Kohlenwasserstoffe, beträgt. In einer Ausführungsform der Erfindung bestehen die Kohlenwasserstoffe der kosmetischen und/oder pharmazeutischen Zubereitung ausschließlich aus linearen C11 und linearen C13 Kohlenwasserstoffen, insbesondere aus n-Undecan und n-Tridecan.

In einer bevorzugten Ausführungsform der Erfindung beträgt das Gewichtsverhältnis von linearen C11-Kohlenwasserstoffen zu linearen C13-Kohlenwasserstoffen in den kosmetischen und/oder pharmazeutischen Zubereitungen 1,5 bis 3,5.

Eine bevorzugte Ausführungsform der Erfindung betrifft kosmetische und/oder pharmazeutische Zubereitungen, enthaltend **1 bis 80 Gew.% Kohlenwasserstoffe,** wobei die Summe der linearen C11-und linearen C13-Kohlenwasserstoffe größer gleich 60 Gew. % bezogen auf die Summe der Kohlenwasserstoffe beträgt und die Summe der **verzweigten** Kohlenwasserstoffe kleiner gleich 10 Gew.-%, bezogen auf die Summe der Kohlenwasserstoffe, beträgt. Besonders bevorzugt sind kosmetische und/oder pharmazeutische Zubereitungen, bei denen die Summe der **verzweigten** Kohlenwasserstoffe kleiner gleich 8 Gew.-%, insbesondere kleiner gleich 5 Gew.%, insbesondere kleiner gleich 3 Gew.-%, bevorzugt kleiner gleich 2 Gew.-% bezogen auf die Summe der Kohlenwasserstoffe, beträgt. In einer bevorzugten Ausführungsform der Erfindung beträgt die Summe der verzweigten Kohlenwasserstoffe kleiner gleich 1 Gew.-% bezogen auf die Summe der Kohlenwasserstoffe.

Eine bevorzugte Ausführungsform der Erfindung betrifft kosmetische und/oder pharmazeutische Zubereitungen, enthaltend **1 bis 80 Gew.% Kohlenwasserstoffe,** wobei die Summe der linearen C11-und linearen C13-Kohlenwasserstoffe größer gleich 60 Gew. % bezogen auf die Summe der Kohlenwasserstoffe beträgt und bei denen die linearen C11 und/oder linearen C13 Kohlenwasserstoffe **gesättigte** Kohlenwasserstoffe sind, vorzugsweise ist sowohl der lineare C11 als auch der lineare C13 Kohlenwasserstoff ein linearer Kohlenwasserstoff (n-Undecan und n-Tridecan).

Eine bevorzugte Ausführungsform der Erfindung betrifft kosmetische und/oder pharmazeutische Zubereitungen, enthaltend **1 bis 80 Gew.% Kohlenwasserstoffe,** wobei die Summe der linearen C11-und linearen C13-Kohlenwasserstoffe größer gleich 60 Gew. % bezogen auf die Summe der Kohlenwasserstoffe beträgt und der Anteil an **C12 Kohlenwasserstoffen** kleiner gleich 10 Gew.-%, insbesondere kleiner gleich 5 Gew.-%, bevorzugt kleiner gleich 3 Gew.-%, bezogen auf die Summe der Kohlenwasserstoffe, beträgt.

Eine bevorzugte Ausführungsform der Erfindung betrifft kosmetische und/oder pharmazeutische Zubereitungen, enthaltend **1 bis 80 Gew.% Kohlenwasserstoffe**, wobei die Summe der linearen C11-und linearen C13-Kohlenwasserstoffe größer gleich 60 Gew. % bezogen auf die Summe der Kohlenwasserstoffe beträgt und wobei die **Summe der Kohlenwasserstoffe mit einer C-Kettenlänge größer gleich 14,** kleiner gleich 15 Gew.-%, insbesondere kleiner gleich 10 Gew.-%, insbesondere kleiner gleich 8 Gew.-%, bevorzugt kleiner gleich 4 Gew.-%, insbesondere kleiner gleich 2 Gew.-% beträgt, jeweils bezogen auf die Summe der Kohlenwasserstoffe, bezogen auf die Summe der Kohlenwasserstoffe, beträgt.

Eine bevorzugte Ausführungsform der Erfindung betrifft kosmetische und/oder pharmazeutische Zubereitungen, enthaltend **1 bis 80 Gew.% Kohlenwasserstoffe**, wobei die Summe der linearen C11-und linearen C13-Kohlenwasserstoffe größer gleich 60 Gew. % bezogen auf die Summe der Kohlenwasserstoffe beträgt und die Summe der **Kohlenwasserstoffe mit einer C-Kettenlänge von kleiner gleich 10,** kleiner gleich 3 Gew.-%, insbesondere kleiner gleich 2 Gew.-%, bevorzugt kleiner gleich 1,5 Gew.-%, insbesondere kleiner gleich 1 Gew.-%, bezogen auf die Summe der Kohlenwasserstoffe, beträgt.

In einer bevorzugten Ausführungsform der Erfindung beträgt das Gewichtsverhältnis von linearen C11-Kohlenwasserstoffen zu linearen C13-Kohlenwasserstoffen 1,5 bis 3,5 in den kosmetischen und/oder pharmazeutischen Zubereitungen.

In einer Ausführungsform der Erfindung enthalten die erfindungsgemäßen kosmetischen und/oder pharmazeutischen Zubereitungen C12 und C14 Kohlenwasserstoffe und zwar im selben Gewichtsverhältnis zueinander wie die linearen C11 Kohlenwasserstoffe zu den linearen C13 Kohlenwasserstoffe. In einer bevorzugten Ausführungsform der Erfindung beträgt sowohl das Gewichtsverhältnis von linearen C11-Kohlenwasserstoffen zu linearen C13-Kohlenwasserstoffen als auch das Gewichtsverhältnis von C12 zu C14 Kohlenwasserstoffen 1,5 bis 3,5.

Eine Ausführungsform der Erfindung betrifft kosmetische und/oder pharmazeutische Zubereitungen, enthaltend **1 bis 80 Gew.% Kohlenwasserstoffe,** wobei die Summe der linearen C11- und linearen C13-Kohlenwasserstoffe größer gleich 60 Gew. % bezogen auf die Summe der Kohlenwasserstoffe beträgt und die Kohlenwasserstoffe aus Kohlenwasserstoffen mit 7 bis 21 Kohlenwasserstoffen, vorzugsweise 9 bis 17 Kohlenwasserstoffen, besonders bevorzugt 11 bis 17 Kohlenwasserstoffen, bestehen.

Mit den erfindungsgemäßen Kohlenwasserstoff Gemischen werden leichte, stabile kosmetische und/oder pharmazeutische Zubereitungen erhalten, dies ist insbesondere dann der Fall, wenn die Kohlenwasserstoff Gemische zusammen mit Antiperspirant-/ Desodorant-Wirkstoffen eingesetzt werden.

Ein Gegenstand der Erfindung betrifft daher kosmetische und/oder pharmazeutische Zubereitungen, enthaltend **1 bis 80 Gew.% Kohlenwasserstoffe,** wobei die Summe der linearen C11- und linearen C13-Kohlenwasserstoffe größer gleich 60 Gew. % bezogen auf die Summe der Kohlenwasserstoffe beträgt und mindestens einen Antiperspirant-/ Desodorant-Wirkstoff.

Erfindungsgemäß sind als **Antiperspirant /Desodorant Wirkstoff** alle Wirkstoffe geeignet, die Körpergerüchen entgegen wirken, diese überdecken oder beseitigen. Körpergerüche entstehen durch die Einwirkung von Hautbakterien auf apokrinen Schweiß, wobei unangenehm riechende Abbauprodukte gebildet werden. Als Antiperspirant /Desodorant Wirkstoffe eignen sich insbesondere Verbindungen ausgewählt aus der Gruppe bestehend Antiperspirantien, Esteraseinhibitoren, bakterizide bzw. bakteriostatische Wirkstoffe und/oder schweißabsorbierende Substanzen.

### Antiperspirantien

Bei Antiperspirantien handelt es sich um Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Vorzugsweise werden Aluminiumchlorhydrat, Aluminium-Zirkonium-tetrachlorohydrat, Aluminium-Zirkonium-pentachlorohydrat und deren Komplexverbindungen eingesetzt.

Die erfindungsgemäßen Zubereitungen können die Antiperspirantien in Mengen von 1 bis 50, vorzugsweise 5 bis 30 und insbesondere 8 bis 25 Gew.-% - bezogen auf das Gesamtgewicht der kosmetischen und/oder pharmazeutischen Zubereitung - enthalten.

### Esteraseinhibitoren

Beim Vorhandensein von Schweiß im Achselbereich werden durch Bakterien extrazelluläre Enzyme - Esterasen, vorzugsweise Proteasen und/oder Lipasen - gebildet, die im Schweiß enthaltene Ester spalten und dadurch Geruchsstoffe freisetzen. Als Esteraseinhibitoren geeignet sind vorzugsweise Trialkylcitrate wie Trimethylcitrat, Tripropylcitrat, Triisopropylcitrat, Tributylcitrat und insbesondere Triethylcitrat (Hydagen® CAT, Cognis GmbH, Düsseldod/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester sowie Zinkglycinat.

Die erfindungsgemäßen Zubereitungen können die Esteraseinhibitoren in Mengen von 0,01 bis 20, vorzugsweise 0,1 bis 10 und insbesondere 0,3 bis 5 Gew.-% - bezogen auf das Gesamtgewicht der kosmetischen und/oder pharmazeutischen Zubereitung - enthalten.

### Bakterizide bzw. bakteriostatische Wirkstoffe

Typische Beispiele für geeignete bakterizide bzw. bakteriostatische Wirkstoffe sind insbesondere Chitosan und Phenoxyethanol. Besonders wirkungsvoll hat sich auch 5-Chlor-2-(2,4-dichlorphenoxy)-phenol erwiesen, das unter der Marke Irgasan® von der Ciba-Geigy, Basel/CH vertrieben wird. Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Famesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Die erfindungsgemäßen Zubereitungen können die bakterizide bzw. bakteriostatische Wirkstoffe in Mengen von 0,01 bis 5 und vorzugsweise 0,1 bis 2 Gew.-% - bezogen auf das Gesamtgewicht der kosmetischen und/oder pharmazeutischen Zubereitung - enthalten.

### Schweißabsorbierende Substanzen

Als schweißabsorbierende Substanzen kommen modifizierte Stärke, wie z.B. Dry Flo Plus (Fa. National Starch), Silikate, Talkum und andere Substanzen ähnlicher Modifikation, die zur Schweißabsorption geeignet erscheinen. Die erfindungsgemäßen Zubereitungen können die schweißabsorbierende Substanzen in Mengen von 0,1 bis 30, vorzugsweise 1 bis 20 und insbesondere 2 bis 8 Gew.-% - bezogen auf das Gesamtgewicht der kosmetischen und/oder pharmazeutischen Zubereitung - enthalten.

Mit den erfindungsgemäßen Kohlenwasserstoff Gemischen werden sensorisch leichte, kosmetische und/oder pharmazeutische Zubereitungen erhalten, dies ist insbesondere dann der Fall, wenn die Kohlenwasserstoff Gemische zusammen mit UV-Lichtschutzfiltem eingesetzt werden.

Ein Gegenstand der Erfindung betrifft daher kosmetische und/oder pharmazeutische Zubereitungen, enthaltend **1 bis 80 Gew.% Kohlenwasserstoffe,** wobei die Summe der linearen C11- und linearen C13-Kohlenwasserstoffe größer gleich 60 Gew. % bezogen auf die Summe der Kohlenwasserstoffe beträgt und mindestens einen **UV-Lichtschutzfilter.**

Erfindungsgemäß sind als **UV-Lichtschutzfilter** bei Raumtemperatur flüssige oder kristalline organische Substanzen (Lichtschutzfilter) geeignet, die in der Lage sind, ultraviolette Strahlen zu absorbieren und die aufgenommene Energie in Form längerwelliger Strahlung, z.B. Wärme wieder abzugeben. UV-Filter können öllöslich oder wasserlöslich sein. Als typische öllösliche UV-B-Filter bzw. Breitspektrum-UV A/B-Filter sind z.B. zu nennen:
➢ 3-Benzylidencampher bzw. 3-Benzylidennorcampher (Mexoryl SDS 20) und dessen Derivate, z.B. 3-(4-Methylbenzyliden)campher wie in der EP 0693471 B1 beschrieben;
➢ 3-(4'-Trimethylammonium) benzyliden- boman-2-on-methylsulfat (Mexoryl SO)
➢ 3,3'-(1,4-Phenylendimethin)-bis (7,7- dimethyl-2-oxobicyclo-[2.2.1] heptan-1-methansulfonsäure) and salts (Mexoryl SX)
➢ 3-(4'-Sulfo)-benzyliden-bornan-2-on and salts (Mexoryl SL)
➢ Polymer von N-{(2und 4)-[2-oxoborn-3-yliden)methyl}benzyl]acrylamid (Mexoryl SW)
➢ 2-(2H-Benzotriazol-2-yl)-4-methyl-6-(2-methyl-3-(1,3,3,3-tetramethyl-1-(trimethylsilyloxy) disiloxanyl)propyl) phenol (Mexoryl XL)
➢ 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoe-säureamylester;
➢ Ester der Zimtsäure, vorzugsweise 4-Methoxyzimtsäure-2-ethylhexylester, 4-Methoxy-zimtsäurepropylester, 4-Methoxyzimtsäureisoamylester, 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-iso-propylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexyl-ester;
➢ Triazinderivate, wie z.B. 2,4,6-Trianilino-(p-carbo-2'-ethyl-1'-hexyloxy)-1,3,5-triazin und 2,4,6-Tris[p-(2-ethylhexyl-oxycar-bonyl) anilino]-1,3,5-triazin (Uvinul T 150) wie in der EP 0818450 A1 beschrieben oder 4,4'-[(6-[4-((1,1-Dimethylethyl)amino-carbonyl) phenyl-amino]-1,3,5-triazin-2,4-diyl)diimino] bis(benzoesäure-2- ethylhexylester) (Uvasorb® HEB);
➢ 2,2(-Methylen-bis(6-(2H-benzotriazol-2-yl)-4- (1,1,3,3-tetramethyl-butyl)phenol) (Tinosorb M);
➢ 2,4-Bis(4-(2-ethylhexyloxy)-2- hydroxyphenyl]-6-(4-methoxyphenyl)-1,3,5- triazin (Tinosorb S);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate, wie in der EP 0694521 B1 beschrieben;
➢ Dimethicodiethylbenzalmalonate (Parsol SLX).

Als wasserlösliche UV - Filter kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ 2,2(-(1,4-Phenylen)bis(1H-benzimidazol- 4,6-disulfon-säure, Mononatriumsalz) (Neo Heliopan AP)
➢ Sulfonsäurederivate von Benzophenonen, vorzugsweise 2-Hydroxy-4-methoxybenzo-phenon-5-sulfonsäure und ihre Salze;
➢ Sulfonsäurederivate des 3-Benzylidencamphers, wie z.B. 4-(2-Oxo-3-bornylidenmethyl)-benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4`-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der DE 19712033 A1 (BASF) sowie Benzoic Acid, 2-[4-(Diethylamino)-2-Hydroxybenzoyl]-, Hexyl Ester (Uvinul® A plus).

Die UV-A und UV-B-Filter können selbstverständlich auch in Mischungen eingesetzt werden. Besonders günstige Kombinationen bestehen aus den Derivaten des Benzoylmethans" z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.
Erfindungsgemäß bevorzugt sind UV-Lichtschutzfilter ausgewählt aus dem Anhang VII der europäischen Kosmetik-Gesetzgebung (24th Adapting Comission Directive, 29.Februar 2000)

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und auch für die dekorative Kosmetik verwendet. Die Partikel sollten einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pig-mente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T, Eusolex® T-2000, Eusolex® T-Aqua, Eusolex® AVO, Eusolex® T-ECO, Eusolex® T-OLEO und Eusolex® T-S (Merck). Typische Beispiele für sind Zinkoxide, wie z.B. Zinc Oxide neutral, Zinc Oxide NDM (Symrise) oder Z-Cote® (BASF) oder SUNZnO-AS und SUNZnO-NAS (Sunjun Chemical Co. Ltd.). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, 543 (1996) sowie Parf.Kosm. 3, 11 (1999) zu entnehmen.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Carnosin und deren Derivate (z.B. Anserin), Carotinoide, Carotine (z.B. -Carotin, -Carotin, Lycopin) und deren Derivate, Chlorogensäure und deren Derivate, Liponsäure und deren Derivate (z.B. Dihydroliponsäure), Auro-thioglucose, Propylthiouracil und andere Thiole (z.B. Thioredoxin, Glutathion, Cystein, Cystin, Cysta-min und deren Glycosyl-, N-Acetyl-, Methyl-, Ethyl-, Propyl-, Amyl-, Butyl- und Lauryl-, Palmitoyl-, Oleyl-, -Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleo-side und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis mol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascor-bylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydro-guajaretsäure, Trihydroxybutyrophenon, Harnsäure und deren Derivate, Mannose und deren Derivate, Superoxid-Dismutase, Zink und dessen Derivate (z.B. ZnO, ZnSO4) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Eine bevorzugte Ausführungsform der Erfindung betrifft kosmetischen und/oder pharmazeutische Zubereitungen, enthaltend **1 bis 80 Gew.% Kohlenwasserstoffe,** wobei die Summe der linearen C11- und linearen C13-Kohlenwasserstoffe größer gleich 60 Gew. % bezogen auf die Summe der Kohlenwasserstoffe beträgt und mindestens einen UV-Lichtschutzfilter ausgewählt aus der Gruppe bestehend aus 4-Methybenzylidene Camphor, Benzophenone-3, Butyl Methoxydibenzoylmethane, Bis-Ethylhexyloxyphenol Methoxyphenyl Triazine, Methylene Bis-Benzotriazolyl Tetramethylbutylphenol, Diethylhexyl Butamido Triazone, Ethylhexyl Triazone und Diethylamino Hydroxybenzoyl Hexyl Benzoate.

Diese UV-Lichtschutzfilter sind beispielsweise unter den folgenden Handelsnamen kommerziell erhältlich: Neo Heliopan MBC (INCl: 4-Methylbenzylidene Camphor; Hersteller: Symrise); Neo Heliopan BB (INCl: Benzophenone-3, Hersteller: Symrise); Parsol 1789 (INCl: Butyl Methoxydibenzoylmethane, Hersteller: Hoffmann-La Roche (Givaudan)); Tinosorb S (INCl: Bis-Ethylhexyloxyphenol Methoxyphenyl Triazine: Hersteller: Ciba Specialty Chemicals Corporation); Tinosorb M (INCl: Methylene Bis-Benzotriazolyl Tetramethylbutylphenol, Herstelller: Ciba Specialty Chemicals Corporation), Uvasorb HEB (INCl: Diethylhexyl Butamido Triazone, Hersteller: 3V Inc.), Uvinul T 150 (INCl: Ethylhexyl Triazone, Hersteller: BASF AG), Uvinul A plus (INCl: Diethylamino Hydroxybenzoyl Hexyl Benzoate, Hersteller: BASF AG).

Die erfindungsgemäßen Zubereitungen können die UV-Lichtschutzfilter in Mengen von 0,5 bis 30 Gew.-%, vorzugsweise 2,5 bis 20 Gew.-%, besonders bevorzugt 5 - 15 Gew.-% - bezogen auf das Gesamtgewicht der kosmetischen und/oder pharmazeutischen Zubereitung- enthalten.

Die erfindungsgemäßen kosmetischen und/oder pharmazeutischen Zubereitungen können beispielsweise als O/W oder W/O Pflegeemulsionen, Sonnenschutzformulierung, AP/Deo Konzepte, Formulierungen für die dekorative Kosmetik, ölige Pflegezubereitungen, Tränkflüssigkeiten für Substrate, wie beispielsweise Papier- und Vliessprodukte vorliegen. Exemplarisch seien genannt Wet Wipes, Taschentücher, Windeln oder Hygieneprodukte.

Die erfindungsgemäßen Kohlenwasserstoff Gemische eignen sich insbesondere auch für leichte sprühbare Anwendungen und/oder als Bestandteile von Pflegeemulsionen für Tissues, Papiere, Wipes, Schwämme (z.B. Polyurethanschwämme), Pflaster im Bereich Baby-Hygiene, Baby-Pflege, Hautpflege, Sonnenschutz, After-SunTreatment, Insektrepellent, Reinigung, Gesichtsreinigung und AP/Deo - Anwendung geeignet sind. Durch den Einsatz der erfindungsgemäßen Kohlenwasserstoff Gemische wird das sensorische Verhalten bei Applikation positiv beeinflusst.

Die kosmetischen und/oder pharmazeutischen Zubereitungen können Formulierungen zur Körperpflege sein, z. B. eine Körpermilch, Cremes, Lotionen, sprühbare Emulsionen, Produkte zur Eliminierung des Körpergeruchs etc. Die Kohlenwasserstoff Gemische lassen sich auch in tensidhaltigen Formulierungen wie z. B. Schaum- und Duschbädem, Haarshampoos und Pflegespülungen einsetzen. Je nach Applikationszweck enthalten die kosmetischen und/oder pharmazeutischen Zubereitungen eine Reihe weiterer Hilfs- und Zusatzstoffe, wie beispielsweise Tenside, weitere Ölkörper, Emulgatoren, Perlglanzwachse, Konsistenzgeber, Verdickungsmittel, Überfettungsmittel, Stabilisatoren, Polymere, Fette, Wachse, Lecithine, Phospholipide, biogene Wirkstoffe, Antischuppenmittel, Filmbildner, Quellmittel, Insektenrepellentien, Selbstbräuner, Tyrosinaseinhibitoren (Depigmentierungsmittel), Hydrotrope, Solubilisatoren, Konservierungsmittel, Parfümöle, Farbstoffe etc., die nachstehend exemplarisch aufgelistet sind.

Als oberflächenaktive Stoffe können anionische, nichtionische, kationische und/oder amphotere bzw. zwitterionische **Tenside** enthalten sein. In tensidhaltigen kosmetischen Zubereitungen, wie beispielsweise Duschgelen, Schaumbädern, Shampoos etc. ist vorzugsweise wenigstens ein anionisches Tensid enthalten. Der Anteil der Tenside liegt hier üblicherweise bei etwa 1 bis 30, vorzugsweise 5 bis 25 und insbesondere 10 bis 20 Gew.-%.

Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside und/ oder deren Gemische mit Alkyloligoglucosidcarboxylaten, Fettsäureglucamide, Alkylamidobetaine, Amphoacetale und/oder Proteinfettsäurekondensate, letztere vorzugsweise auf Basis von Weizenproteinen bzw. deren Salzen.

Körperpflegemittel, wie Cremes, Lotionen und Milchen, enthalten üblicherweise eine Reihe **weiterer Ölkörper** und Emollients, die dazu beitragen, die sensorischen Eigenschaften weiter zu optimieren. Die Ölkörper können, ja nach Art der Formulierung in einer Gesamtmenge von 1 bis 90 Gew.-%, insbesondere in einer Gesamtmenge von 1 - 50 Gew.-%, vorzugsweise 5 - 25 Gew.-% und insbesondere 5 - 15 Gew.-% enthalten sein. Als weitere Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z. B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Feftalkoholcarbonate, wie z. B. Dica-prylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Finsolv® TN), lineare oder verzweigte, symmetrische oder unsymmetrische Dialkylether mit 6 bis 22 Kohlenstoffatomen pro Alkylgruppe, wie z. B. Dicaprylyl Ether (Cetiol® OE), Ringöffnungsprodukte von epoxidierten Fettsäureestern mit Polyolen.

**Fette und Wachse** werden den Körperpflegeprodukten als Pflegestoffe zugesetzt und auch, um die Konsistenz der Kosmetika zu erhöhen. Typische Beispiele für Fette sind Glyceride, d. h. feste pflanzliche oder tierische Produkte, die im Wesentlichen aus gemischten Glycerinestern höherer Fettsäuren bestehen. Auch Fettsäurepartialglyceride, d. h. technische Mono- und/oder Diester des Glycerins mit Fettsäuren mit 12 bis 18 Kohlenstoffatomen wie etwa Glycerinmono/dilaurat, -palmitat oder -stearat kommen hierfür in Frage. Als Wachse kommen u. a. natürliche Wachse, wie z. B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z. B. Montanesterwachse, Sasolwachse, hydrierte Jojobawachse sowie synthetische Wachse, wie z. B. Polyalkylenwachse und Polyethylenglycolwachse in Frage. Neben den Fetten kommen als Zusatzstoffe auch fettähnliche Substanzen, wie Lecithine und Phospholipide in Frage. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Als Verdickungsmittel eignen sich beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, Polyvinylalkohol, Polyvinylpyrrolidon und Bentonite wie z. B. Bentone® Gel VS-5PC (Rheox) sowie Polyacrylate sowie polymere quarternäre Ammoniumsalze, wie beispielsweise Polymere mit der INCI Bezeichnung Polyquatemium-11, Polyquaternium-13, Polyquaternium-14, Polyquatemium-15, Polyquaternium-28, Polyquaternium-32, Polyquaternium-43, Polyquaternium-47.

Unter **biogenen Wirkstoffen** sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

Als **Insekten-Repellentien** kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent^{®} 3535 von der Merck KGaA vertrieben wird, sowie Butylacetylaminopropionate in Frage.

Als **Selbstbräuner** eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

Als **Konservierungsmittel** eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Als **Parfümöle** seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

Als **Perlglanzwachse,** insbesondere für den Einsatz in tensidischen Formulierungen, kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Stabilisatoren** können Metallsalze von Fettsäuren, wie z. B. Magnesium-, Aluminium- und/oder Zinkstearat bzw. -ricinoleat eingesetzt werden.

Zur Verbesserung des Fließverhaltens können ferner **Hydrotrope**, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein.

### Beispiele

### Herstellbeispiel 1: Herstellung eines erfindungsgemässen Kohlenwasserstoff Gemischs

Zur Herstellung eines erfindungsgemäßen Kohlenwasserstoff Gemischs wurden zunächst Tridecan und Undecan getrennt voneinander aus den jeweiligen Fettalkoholen hergestellt und dann im gewünschten Verhältnis zueinander gemischt:

### 1a) Herstellung von Tridecan aus 1-Tetradecanol

1000 g 1-Tetradecanol (4,7 mol; Lorol C 14 der Fa. Cognis) wurden in einem rührbaren Druckbehälter mit 10 g eines Nickelkatalysators (Ni-5249 P der Fa. Engelhard; Ni-Gehalt = 63 Gew.-%) vorgelegt und auf 240°C aufgeheizt. Anschließend wurde über einen Zeitraum von 12 h Wasserstoff über ein Begasungsrohr bei einem Druck von 20 bar zugegeben und gleichzeitig über ein Ventil am Reaktordeckel die Reaktionsgase ausgeschleust. Danach wurde das Produkt gekühlt, abgelassen und filtriert. Es ergab sich eine Auswaage von 845 g Reaktionsprodukt.

Eine GC-Analyse ergibt folgende Zusammensetzung: 89,0 % Tridecan, 2,1 % Tetradecan, 4,1 % 1-Tetradecanol, 4,2 % Dimere Reaktionsprodukte. Dieses Reaktionsprodukt wurde in einer Destillation zum reinen Tridecan fraktioniert und anschließend mit Stickstoff desodoriert. Man erhält ein farbloses, dünnflüssiges und geruchsarmes Produkt.

### 1b) Herstellung von Undecan aus 1-Dodecanol

1000 g 1-Dodecanol (5,4 mol; Lorol C 12 der Fa. Cognis) wurden in einem rührbaren Druckbehälter mit 10 g eines Nickelkatalysators (Ni-5249 P der Fa. Engelhard; Ni-Gehalt = 63 Gew%) vorgelegt und auf 240°C aufgeheizt. Anschließend wurde über einen Zeitraum von 8 h Wasserstoff über ein Begasungsrohr bei einem Druck von 20 bar zugegeben und gleichzeitig über ein Ventil am Reaktordeckel die Reaktionsgase ausgeschleust. Danach wurde das Produkt gekühlt und abgelassen und filtriert. Es ergab sich eine Auswaage von 835g Reaktionsprodukt.

Eine GC-Analyse ergibt folgende Zusammensetzung: 68,4 % Undecan, 0,6 % Dodecan, 21,7 % 1-Dodecanol, 7,2 % Dimere Reaktionsprodukte. Dieses Reaktionsprodukt wurde destilliert, um das Undecan rein zu erhalten. Dieses wurde anschließend mit Stickstoff desodoriert. Man erhält ein farbloses, dünnflüssiges und geruchsarmes Produkt.

Aus den gemäß Beispiel 1a) sowie gemäß Beispiel 1b) erhaltenen Verbindungen wurde folgendes erfindungsgemäßes Kohlenwasserstoff Gemisch hergestellt:
Zusammensetzung des Kohlenwasserstoff Gemischs nach Beispiel 1: 76 Gew.-% n-Undecan, 24 Gew.-% n-Tridecan.

### Herstellbeispiel 2

Zur Herstellung eines erfindungsgemäßen Kohlenwasserstoff Gemischs wurde ein Fettalkohol Gemisch, welches C12 und C14 Fettalkohole entsprechend dem herzustellenden Kohlenwasserstoff Gemisch enthält,einer reduktiven Dehydroxymethylierung unterzogen.

1000 g Lorol® Spezial (Fa. Cognis; Fettalkoholverteilung C 12 70-75 %, C 14 24-30%. C16 unter 4%) und 10 g eines Nickelkatalysators (Ni-5249 P der Fa. Engelhard; Ni-Gehalt = 63 Gew.-%) wurden im Autoklaven vorgelegt. Der Reaktor wurde verschlossen und evakuiert. Das Reaktionsgemisch wurde anschließend unter Vakuum auf ca. 80°C erhitzt und 30 min. gerührt. Danach wurde der Reaktor mit Wasserstoff auf ca. 80 bar gebracht und kontinuierlich auf 250°C erhitzt. Die Reaktion ist abgeschlossen, wenn der Druck konstant bleibt und die Hauptmenge des Fettalkohols zum gewünschten Kohlenwasserstoff umgesetzt ist. Nach Entspannen und Belüften mit Stickstoff wurde das Produkt destillativ gereinigt. Das gereinigte Produkt fällt als farblose Flüssigkeit an.

Das gemäß Herstellbeispiel 2 erhältliche Kohlenwasserstoff Gemisch ist wie folgt zusammensetzt: (GC Analyse)

| Kohlenwasserstoffe mit einer C Zahl von | Anteil ans Gesamtsumme der Kohlenwasserstoffe [Gew.-%] |
|---|---|
| C11 (linear) | 67 |
| C12 | 4 |
| 13 (linear) | 26 |
| C14 | 1,5 |
| C9, C10, C15 | 1,5 |

Das Gewichtsverhältnis von lineare C11 Kohlenwasserstoff zu linearen C13 Kohlenwasserstoff beträgt 2,57. Ebenso beträgt das Gewichtsverhältnis der C12 Kohlenwasserstoffe zu den C14 Kohlenwasserstoffen 2,57

### Rezepturbeispiele

Das gemäß Herstellbeispiel 1 sowie das gemäß Herstellbeispiel 2 erhaltene Kohlenwasserstoff Gemisch wurde in den folgenden kosmetischen Rezepturen eingesetzt.

### Allzweck Creme (W/O)

| Phase | Komponente | INCI | Gew.% |
|---|---|---|---|
| | Handelsname | | |
| I. | DEHYMULS® E | Dicocoyl Pentaerhytithyl | |
| | | Distearyl Citrate (and) Sorbitan | |
| | | Sesquioleate (and) Cera Alba | |
| | | (Beeswax) (and) Aluminum Stearate | 3,00 |
| | | Polyglyceryl | 2 |
| | DEHYMULS® PGPH | Dipolyhydroxystearate | 2,00 |
| | CETIOL® OE | Dicaprylyl Ether | 5,00 |
| | CETIOL® 868 | Ethylhexyl Stearate | 5,00 |
| | MYRITOL® 331 | Cocoglycerides | 1,00 |
| | Kohlenwasserstoff Gemisch gemäß | | |
| | Herstellbeispiel 1 oder Herstellbeispiel 2 | | 6,00 |
| II. | Glycerin 86% | | 5,00 |
| | MgSO₄×7H₂O | | 1,00 |
| | Wasser, deionisiert | | 72,00 |
| III. | Konservierungsmittel | | q.s. |

### Herstellung:

Die Komponenten der Phase I wurden bei 80 bis 85 °C geschmolzen und zur Homogenität verrührt. Die Komponenten der Phase II wurden auf 80 bis 85 °C erhitzt und langsam, unter Rühren zur Phase I zugegeben. Es wurde für weitere 5 Minuten bei dieser Temperatur gerührt. Danach wurde die Emulsion unter Rühren abgekühlt und bei 65 bis 55 °C homogenisiert. Sobald die Emulsion homogen erscheint, wurde unter Rühren weiter auf 30 °C abgekühlt. Danach wurden Komponenten der Phase III zugegeben und erneut gerührt.

### Balsam zur Befeuchtung und zum Schutz der Lippen

| Phase | Komponente | INCl | Gew.-% |
|---|---|---|---|
| | Handelsname | | |
| I. | Cerilla Raffinée G* | Candelilla (Euphorbia Cerifera) Wax | 7,53 |
| | CUTINA® LM conc. | Polyglyceryl-2 Dipolyhydroxystearate and | |
| | | Octyldodecanol and Copemicia Cerifera | |
| | | (Camauba) Wax and Euphorbia Cerifera | |
| | | (Candelilla) Wax and Beeswax and Cetearyl | |
| | | Glucoside and Cetearyl Alcohol | 6,57 |
| | Colophane claire type Y | Rosin | 1,89 |
| | Cerauba T1* | Camauba(Copemica Cerifera)Wax | 1,86 |
| | Cerabeil blanche 1* | Beeswax | 5.31 |
| | Kohlenwasserstoff-Gemisch gemäß Herstellbeispiel 1 oder 2 | | 15.57 |
| | EUTANOL®G | Octyldodecanol | 21,71 |
| | Crodamol ML(Croda) | Myristyl Lactate | 1,13 |
| | ELESTAB®366 | | 0.43 |
| II. | Castor oil | Castor Oil | 35.00 |
| III. | IRWINOL® LS 9319 | African wild mango butter | 3.00 |

| | | | |
|---|---|---|---|
| * erhältlich von Lambert- Rivière (France) | | | |

Herstellung: Phase I wurde bei 85°C geschmolzen, Phase II wurde zugefügt und die Temperatur auf 80°C gehalten. Phase III wurde kurz vor dem Einfüllen in die Gießform (welche mit Dimethicon 50 cts befeuchtet und auf 40 °C vorgeheizt war) zugegeben. Die Masse wurde in die Gießform gegeben und auf 40 °C abgekühlt. Die Gießformen wurde im Gefrierschrank auf nahe 0 °C abgekühlt.

### Styling Wachs

| Phase | Komponente | lNCl | Gew.-% |
|---|---|---|---|
| | Handelsname | | |
| I. | CUTINA® MD | Glyceryl Stearate | 47,0 |
| | COMPERLAN® 100 | Cocamide MEA | 2,50 |
| | CUTINA® HR Powder | Hydrogenated Castor Oil | 2,50 |
| | PLANTACARE® 1200 | | |
| | UP | Lauryl Glucoside | 5,00 |
| | LANETTE® O | Cetearyl Alcohol | 7,00 |
| | CUTINA® CP | Cetyl Palmitate | 7,00 |
| | EUMULGIN® O 20 | Oleth-20 | 5,00 |
| | Kohlenwasserstoff Gemisch gemäß | | |
| | Herstellbeispiel 1 oder 2 | | 23,5 |
| | Wacker Siliconoil AK 350 | Dimethicone | 0,50 |

Die Herstellung erfolgt durch Erhitzen aller Komponenten auf 80 °C und Homogenisierung.

### Feuchtigkeitsspendende Körpermilch

| Phase | Komponente | INCI | |
|---|---|---|---|
| | Handelsname | | Gew.-% |
| | | Cetearyl Isononanoate (and) Ceteareth-20 (and) | |
| I. | EMULGADE® CM | Cetearyl Alcohol (and)Glyceryl Stearate (and) | |
| | | Glycerin (and) Ceteareth | 5.0 |
| | EUMULGIN® VL 75 | Lauryl Glucoside (and) Polyglyceryl-2 | |
| | | Dipolyhydroxystearate (and) Glycerin | 2.0 |
| | CETIOL® OE | Dicaprylyl Ether | 4.0 |
| | CETIOL® J 600 | Oleyl Erucate | 1.0 |
| | ISOPROPYLMYRISTATE | Isopropyl Myristate | 7.0 |
| | Kohlenwasserstoff Gemisch gemäß Herstellbeispiel 1 oder 2 | | 7.0 |
| II. | Wasser, deionisiert | | ad 100 |
| III. | Cosmedia SP | Sodium Polyacrylate | 0.4 |
| IV. | HISPAGEL® 200 | Glycerin (and) Glyceryl Polyacrylate | 20.0 |
| V. | Konservierungsmittel, Parfum | | q.s. |
| | pH | 5.5 | |

Die Herstellung erfolgte durch Mischen von Phase I und Wasser bei Raumtemperatur unter Rühren. Dann wurde Phase III zugefügt und solange gerührt bis eine homogene, gequollene Mischung vorlag. Dann wurde Phase IV zugefügt, gefolgt von Phase V, danach wurde der pH Wert eingestellt.

### O/W Soft Creme

| Phase | Komponente | INCI | |
|---|---|---|---|
| | Handelsname | | Gew.-% |
| | EMULGADE® SE-PF | Glyceryl Stearate (and) Ceteareth-20 (and) Ceteareth- | |
| | | 12 (and) Stearyl Alcohol (and) Ceteareth-20 (and) Distearyl Ether | 6.0 |
| | LANETTE® O | Cetearyl Alcohol | 1.0 |
| | CUTINA® MD | Glyceryl Stearate | 2.0 |
| | CETIOL® MM | Myristyl Myristate | 2.0 |
| | Kohlenwasserstoff Gemisch gemäß Herstellbeispiel 1 oder 2 | | 8.0 |
| | Jojoba Oil | Simmondsia Chinensis (jojoba) Seed Oil | 2.0 |
| | COPHEROL® 1250 | Tocopheryl Acetate | 0.5 |
| | | | |
| | | Dimethicone | 0.5 |
| | | Cyclomethicone | 3.0 |
| II. | Wasser | Aqua | ad 100 |
| | | Propylene Glycol | 3.0 |
| III. | HISPAGEL® 200 | Glycerin (and) Glyceryl Polyacrylate | 15.0 |
| IV. | Konservierungsmittel | | q.s. |
| | pH | 5.5-6.5 | |

Diese Creme wurde hergestellt, indem Phase I auf 80°C erhitzt wurde, Phase II wurde ebenfalls auf 80°C erhitzt und zu Phase I unter Rühren hinzugefügt. Diese Mischung wurde unter Rühren abgekühlt und bei ca. 55°C mit einem geeigneten Dispersionsgerät (z.B. Ultra Turrax) homogenisiert. Danach wurde Phase III unter kontinuierlichem Rühren hinzugefügt, Phase IV wurde zugegeben und der pH-Wert eingestellt.

### W/O Creme

| Phase | Komponente | INCl | Gew.-% |
|---|---|---|---|
| | Handelsname | | |
| I. | MONOMULS® 90 O 18 | Glyceryl Oleate | 2,00 |
| | | | |
| | LAMEFORM® TGI | Polyglyceryl 3 Diisostearate | 4,00 |
| | CETIOL® A | Hexyl Laurate | 12,00 |
| | Kohlenwasserstoff Gemisch gemäß Herstellbeispiel 1 oder 2 | | 12,00 |
| | SIPOL® C 16/18 OR | Cetearyl Alcohol | 1,00 |
| | Zinc stearate | Zinc Stearate | 2,00 |
| | Zinc Oxide | Cl 77947 (or) Zinc Oxide | 15,00 |
| | Magnesium Sulphate | Magnesium Sulphate | 1,00 |
| | Glycerin | Glycerin | 3,00 |
| | Konservierungsmittel | | q.s. |
| | Benzyl Alcohol | Benzyl Alcohol | 0,40 |
| | HYDAGEN® B | Bisabolol | 0,50 |
| | Wasser | aqua | 100,00 |

Die ersten 7 Komponenten wurden bei 85°C geschmolzen. Magnesium Sulfate und Glycerin wurden im Wasser gelöst und diese Mischung wurde auf 85 °C erhitzt. Diese wässrige Phase wurde zur Ölphase gegeben und dispergiert. Unter kontinuierlichen Rühren wurde bis auf 40°C abgekühlt und dann wurden Benzyl Alkohol und Hydagen B gemischt und zu der Emulsion gegeben. Unter weiterem Rühren wurde bis auf 30°C abgekühlt und homogenisiert.

### "Body Wash" Reinigungsemulsion

| Phase | Komponente | INCI | |
|---|---|---|---|
| | Handelsname | | Gew.-% |
| I. | Texapon ALS-IS | Ammonium Lauryl | |
| | | Sulfate | 30,00 |
| | TEXAPON® NSO | Sodium Laureth Sulfate | 18,00 |
| | | | |
| | Kohlenwasserstoff Gemisch gemäß | Herstellbeispiel 1 oder 2 | 18,00 |
| | Plantacare® 1200 | Lauryl Glucoside | 8,00 |
| II. | Jaguar HP 105 | Hydroxypopyl Guar | 2,00 |
| | | Methyldibromo | |
| | | Glutaronitrile and | |
| | Euxyl K400 | Phenoxyethanol | 0,10 |
| | Wasser | Aqua | 23,90 |
| | pH-value | 5,6 | |

### Tabelle 1: O/W-Sonnenschutzemulsionen

Die im Folgenden genannten Beispiele enthalten alle das gemäß Herstellbeispiel 1 oder gemäß Herstellbeispiel 2 erhaltene Kohlenwasserstoff Gemisch. Alle Angaben sind in Gew.-%.

| **Komponente** | **1** | **2** | **3** | **4** | **5** | **6** | **7** | **8** | **9** | **10** | **11** |
|---|---|---|---|---|---|---|---|---|---|---|---|
| **L = Lotion, C = Creme,** | **L** | **C** | **S** | **L** | **C** | **L** | **L** | **C** | **L** | **C** | **L** |
| Eumulgin^{®} VL 75 | | | | | | 4 | 4 | 2 | | | |
| Eumulgin^{®} B2 | 2 | | | | | | | | | | |
| Tween^{®} 60 | | | | 1 | | | | | | | |
| Myrj^{®} 51 | | 3 | | 2 | | | | | | | |
| Cutina^{®} E 24 | 1 | | | 1 | | | | | | | |
| Hostaphat^{®} KL 340 N | | | | | | | | | 2 | | |
| Lanette^{®} E | | | 0.5 | | | | | 0.5 | | | |
| Amphisol^{®} K | | | 1 | | | 1 | | 0.5 | | 1 | |
| Natriumstearat | | | | | | | 1 | | | | 2 |
| Emulgade^{®} PL 68/50 | | | 1 | | 5 | | | | | 4 | |
| Tego^{®} Care 450 | | | | | | | | | | 3 | |
| Cutina^{®} MD | 2 | | | 6 | | | 4 | | | 6 | |
| Lanette^{®} 14 | 1 | | | 1 | | | | 2 | | | 4 |
| Lanette^{®} O | 1 | 6 | | | 5 | 2 | | 2 | | | |
| Antaron V 216 | | | 1 | | 2 | 2 | | | | 1 | |
| Lanolin, wasserfrei USP | | | | | | | 5 | | | | |
| Kohlenwasserstoff-Gemisch nach Herstellbsp. 1 od. 2 | 2 | 2 | 4 | 1 | 2 | 2 | 2 | 1 | 2 | 2 | 1 |
| Myritol^{®} PC | | | | | 5 | | | | | | |
| Myritol^{®} 331 | 5 | | 8 | | | 6 | | 10 | | 2 | |
| Finsolv^{®} TN | | | 1 | | | | | 1 | 8 | | |
| Cetiol^{®} CC | | 2 | 5 | | | 4 | 4 | 2 | | 2 | |
| Cetiol^{®} OE | | | 3 | | | | | | 2 | 3 | |
| Dow Corning DC^{®} 244 | 4 | | 1 | | 5 | | | 2 | | | 2 |
| Dow Corning DC^{®} 2502 | | 1 | | | 2 | | | | | | |
| Silikonöl Wacker AK^{®} 350 | | 2 | | | | | | | | | |
| Cetiol^{®} 868 | | | | | 2 | | 8 | | | | 7 |
| Cetiol^{®} J 600 | | | | | 3 | 2 | | | | 5 | |
| Mineralöl | | | | 9 | | | | | | | |
| Cetiol^{®} B | | | 1 | | | | | | | 2 | |
| Eutanol^{®} G | | | | | | | | | | | |
| Eutanol^{®} G 16 | | | | | | | | | | | |
| Cetiol^{®} PGL | | 5 | | | | | | | | 5 | |
| Mandelöl | | | 2 | | | | 1 | | | | |
| Photonyl^{®} LS | | | | 2 | | | | | | 2 | |
| Panthenol | 1 | | | | | | | | | | |
| Bisabolol | 0.2 | | | | | | | | | | |
| Tocopherol / Tocopherylacetat | 1 | | | | | | | | | | |
| Photonyl^{®} LS | | | | | | | | | | | |
| Neo Heliopan^{®} Hydro (Na-Salz) | 2 | | 2.2 | | 3 | 3 | | | | | 2 |
| Neo Heliopan AP (Na-Salz) | 2 | | | | 1,5 | 2 | 2 | | 1 | | 1 |
| Neo Heliopan^{®} 303 | 3 | 5 | 9 | 4 | | | | | | | |
| Neo Heliopan^{®} BB | | | | | 1 | | | | | | 2 |
| Neo Heliopan^{®} MBC | 2 | | | 3 | | 2 | 2 | 2 | | | 1 |
| Neo Heliopan^{®} OS | | | | | | | | | 10 | 7 | |
| Neo Heliopan^{®} E 1000 | | 7.5 | | 6 | | | | | | | 6 |
| Neo Heliopan^{®} AV | | | 7.5 | | | 7.5 | 4 | 5 | | | |
| Uvinul^{®} T 150 | 2 | | | | 2.5 | | | 1 | | | |
| Parsol^{®} 1789 | | 1 | 1 | | | | 2 | | 2 | 2 | |
| Zinkoxid NDM | 10 | | 5 | | | 10 | | 3 | | 5 | 4 |
| Eusolex® T 2000 | | | | | 5 | | 3 | 3 | | | 4 |
| Veegum^{®} Ultra | | | 0.75 | | | | | 1 | 1 | | |
| Keltrol^{®} T | | | 0.25 | | | | | 0.5 | 0.5 | | |
| Carbopol^{®} 980 | | 0.5 | | 0.2 | 0.2 | 0.2 | | 0.5 | 0.1 | 0.3 | 0.2 |
| Ethanol | | | | | | | | | | 10 | |
| Butylenglykol | | 2 | | 4 | 3 | | 2 | 5 | 2 | | 2 |
| Glycerin | 5 | 5 | 5 | | 3 | 3 | 2 | | 4 | | 3 |
| Konservierungsmittel, NaOH | q.s. | | | | | | | | | | |
| Wasser | ad 100 | | | | | | | | | | |

**Tabelle 2: O/W-Sonnenschutzemulsionen**

| **Komponente** | **12** | **13** | **14** | **15** | **16** | **17** | **18** | **19** | **20** | **21** | **22** |
|---|---|---|---|---|---|---|---|---|---|---|---|
| **L = Lotion, C = Creme,** | **L** | **L** | **L** | **C** | **L** | **C** | **S** | **C** | **C** | **L** | **L** |
| Eumulgin^{®} VL 75 | 4 | 3 | 4.5 | | 3 | | | | 4 | | |
| Eumulgin^{®} B2 | | | | | | | | | | 1 | |
| Tween^{®} 60 | | | | | | | | | | 1 | |
| Myrj^{®} 51 | | | | | | | | | | | |
| Cutina^{®} E 24 | | | | 2 | | | | | | | |
| Hostaphat^{®} KL 340 N | | | | | | | | | | 0.5 | |
| Lanefte^{®} E | 0.5 | | 0.5 | 0.5 | | | 0.1 | | 0.5 | | |
| Amphisol^{®} K | 0.5 | | | | | 1 | 1 | 1 | | | |
| Natriumstearat | | | | | 1 | | | | | | |
| Emulgade^{®} PL 68/50 | | 6 | | | | 4.5 | 1 | 5 | | | |
| Tego^{®} Care 450 | 1 | | | | | | | | 4 | | |
| Cutina^{®} MD | 1 | | | 8 | 6 | 1 | | | | 4 | 1 |
| Lanette^{®} 14 | | 2 | | | | | | 2 | | 1 | |
| Lanette^{®} O | | | | 2 | | | | | 1 | 1 | |
| Antaron V 220 | 1 | | | 2 | | | 0.5 | | | 2 | 0.5 |
| Kohlenwasserstoff-Gemisch nach Herstellbsp.1 od. 2 | 4 | 2 | 4 | 6 | 10 | 4 | 2 | 8 | 2 | 1 | 3 |
| Myritol^{®} PC | | | | | | | | | 5 | | |
| Myritol^{®} 331 | 12 | | 12 | | | 8 | 8 | | | 10 | 8 |
| Finsolv^{®} TN | | | | | 5 | | | 3 | 3 | | |
| Cetiol^{®} CC | 6 | | 6 | | | 5 | 5 | | | | |
| Cetiol^{®} OE | | | | | 2 | | | | | | 2 |
| Dow Corning DC^{®} 244 | | 2 | | | 1 | | | | | | |
| Dow Corning DC^{®} 2502 | | 1 | | | 1 | | | | | | |
| Ceraphyl^{®} 45 | | | | | | | | | | 2 | 2 |
| Silikonöl Wacker AK^{®} 350 | | | | | 1 | | | | | | |
| Cetiol^{®} 868 | | 2 | | | | | | | | | |
| Cetiol^{®} J 600 | | 2 | | | | | | | | | |
| Cetiol^{®} B | 4 | | 4 | | | | | 4 | | | |
| Eutanol^{®} G | | 3 | | 5 | | 3 | | | | | |
| Eutanol^{®} G 16 S | 10 | | | | | | | | | | |
| Cetiol^{®} PGL | | | | 5 | | | | | 2 | | |
| Photonyl^{®} LS | | | | | | | | | | 2 | |
| Panthenol | 1 | | | | | | | | | | |
| Bisabolol | 0,2 | | | | | | | | | | |
| Tocopherol / Tocopherylacetat | 1 | | | | | | | | | | |
| Neo Heliopan^{®} Hydro (Na-Salz) | | | | | | | | | | 3 | |
| Neo Heliopan AP (Na-Salz) | | 2 | | 2 | | | 2 | | | | 1 |
| Eusolex^{®} OCR | 6 | | 9 | | 5 | 7 | 9 | | 4 | | 7 |
| Neo Heliopan^{®} BB | | | | | | | | 1 | 1 | | 1 |
| Neo Heliopan^{®} MBC | | 2 | | 1 | | | | 3 | 1 | | 3 |
| Neo Heliopan^{®} OS | 2 | | | | | | | | 7 | | |
| Neo Heliopan^{®} E1000 | | 4 | | | | | | 5 | | | |
| Neo Heliopan^{®} AV | | 4 | 7.5 | 5 | | | | 5 | 4 | 7.5 | |
| Uvinul^{®} T 150 | 1 | | | | | | | | 1.3 | 1 | 1 |
| Parsol^{®} 1789 | 1 | | | | | | | | 2 | | 1 |
| Z-Cote^{®} HP 1 | 7 | 2 | 5 | | | 7 | 5 | | 6 | 2 | |
| Eusolex^{®} T 2000 | 5 | 2 | | | 10 | | | 10 | | 2 | |
| Veegum^{®} Ultra | 1.5 | | 1.5 | | | 1.5 | 1.2 | | 1 | | |
| Keltrol^{®} T | 0.5 | | 0.5 | | | 0.5 | 0.4 | | 0.5 | | |
| Pemulen^{®} TR 2 | | 0.3 | | 0.3 | | | 0.1 | 0.2 | | | 0.3 |
| Ethanol | | 5 | | 8 | | | | | | | |
| Butylenglykol | 1 | | | 3 | 3 | | | | | 8 | 1 |
| Glycerin | 2 | 4 | 3 | 3 | | 3 | 3 | 3 | 5 | | 3 |
| Wasser/ Konservierungsmittel/ NaOH | ad 100/ q.s./ q.s | | | | | | | | | | |

**Tabelle 3: W/O-Sonnenschutzemulsionen**

| **Komponente** | **23** | **24** | **25** | **26** | **27** | **28** | **29** | **30** | **31** | **32** | **33** |
|---|---|---|---|---|---|---|---|---|---|---|---|
| **L = Lotion; C = Creme** | **C** | **L** | **C** | **L** | **C** | **L** | **L** | **L** | **L** | **C** | **C** |
| Dehymuls^{®} PGPH | 4 | 2 | 1 | 3 | 3 | 1 | 1 | 2 | 2 | 4 | 1 |
| Monomuls^{®} 90-O18 | | | 2 | | | | | | | | |
| Lameform^{®} TGI | 2 | | 4 | | 3 | | | | | 1 | 3 |
| Abil^{®} EM 90 | | | | | | | 4 | | | | |
| Glucate^{®} DO | | | | | | | | | | | 3 |
| Isolan^{®} PDI | | | | | | 4 | | 2 | | | |
| Arlacel^{®} 83 | | | | 2 | | | | | | | |
| Elfacos^{®} ST9 | | | | | | | | | 2 | | |
| Elfacos^{®} ST37 | | | | | | | | | | | |
| Arlacel^{®} P 135 | | 2 | | | | | | | | | |
| Dehymuls^{®} HRE 7 | | | | | | | | | | | |
| Zinkstearat | 1 | | | 1 | 1 | | | 1 | | 1 | |
| Bienenwachs | 1 | | 2 | 1 | | 1 | | 3 | | 2 | 3 |
| Tego^{®} Care CG | | | | | 1 | | | | | | .5 |
| Prisorine^{®} 3505 | 1 | | 1 | 1 | | 1 | 1 | | | | 1 |
| Wollwachsalkohol, wasserfrei, USP | | | | | | | | | | | 1 |
| Antaron V 216 | 2 | | | | | | | | | | |
| Kohlenwasserstoff-Gemisch nach Herstellbsp. 1 od. 2 | 3 | 4 | 2 | 1 | 10 | 2 | 2 | 6 | 3 | 12 | 1 |
| Myritol^{®} PC | | | | | 3 | | | 4 | | | |
| Myritol^{®} 331 | 10 | | | | 3 | 6 | | | | | 8 |
| Finsolv^{®} TN | | | | 5 | | | 5 | | | | |
| Cetiol^{®} CC | 12 | 22 | | | | 2 | | | 2 | | 5 |
| Cetiol^{®} OE | | | | | 4 | | 5 | | 4 | 2 | |
| Dow Corning DC^{®} 244 | | | | | | | 2 | | | | |
| Dow Corning DC^{®} 2502 | | | 1 | | 2 | | | | | | |
| Prisorine^{®} 3758 | | | | | | | | | | 2 | |
| Silikonöl Wacker AK^{®} 350 | | | | 4 | | | | 3 | | | |
| Cetiol^{®} 868 | | | | | | | | | | 2 | |
| Eutanol^{®} G 16 | | 3 | | | | | | | | | |
| Eutanol^{®} G 16S | | | | | | | | | | | |
| Cetiol^{®} J 600 | | | 4 | | | 2 | | | | | |
| Ceraphyl^{®} 45 | | | | 2 | | | | 2 | | 6 | |
| Cetiol^{®} B | | | 2 | 4 | | | | | | 3 | |
| Eutanol^{®} G | | | 3 | | 2 | | | 8 | | | |
| Cetiol^{®} PGL | | 11 | | | 2 | 4 | | | 9 | | |
| Mandelöl | | | | | 1 | | 5 | | | | |
| Photonyl^{®} LS | | 2 | | 1 | | | | | 4 | | |
| Panthenol | 1.0 | | | | | | | | | | |
| Bisabolol | 0.2 | | | | | | | | | | |
| Tocopherol / Tocopherylacetat | 1,0 | | | | | | | | | | |
| Magnesiumsulfat x 7 Wasser | 1 | | | | | | | | | | |
| Neo Heliopan^{®} Hydro (Na-Salz) | | 2 | | 3 | | | | 2 | | | |
| Neo Heliopan AP (Na-Salz) | 2 | 1 | | | 2 | | | 1 | 2 | | 1 |
| Neo Heliopan^{®} 303 | | | | | 4 | | | | | 6 | |
| Neo Heliopan^{®} BB | | 4 | 2 | | | | 2 | | | | |
| Neo Heliopan^{®} MBC | | | | | | | | 4 | | 3 | |
| Neo Heliopan^{®} OS | | | | | | | | | | | |
| Neo Heliopan^{®} E 1000 | | | | | | | | | 5 | | |
| Neo Heliopan^{®} AV | | 3 | 6 | 6 | | 7.5 | 7.5 | | 5 | | 7.5 |
| Uvinul^{®} T 150 | | | | | 2.5 | | | 1 | | 2 | |
| Parsol^{®} 1789 | | 2 | | | | | | 1 | | 2 | |
| Zinkoxid NDM | | | | | | 6 | | | | | |
| Eusolex^{®} T 2000 | 15 | | 10 | | 5 | | 4 | | | | 4 |
| Ethanol | | | | | | | | | | 8 | |
| Butylenglykol | | | 2 | 6 | | | 2 | 5 | | | 2 |
| Glycerin | 5 | 3 | 3 | | 5 | 3 | 2 | | 10 | 4 | |
| Wasser, Konservierungsmittel | ad 100, q.s. | | | | | | | | | | |

**Tabelle 4: W/O-Sonnenschutzemulsionen**

| **Komponente** | **34** | **35** | **36** | **37** | **38** | **39** | **40** | **41** | **42** | **43** | **44** |
|---|---|---|---|---|---|---|---|---|---|---|---|
| **L = Lotion; C = Creme** | **L** | **C** | **L** | **L** | **C** | **L** | **L** | **L** | **L** | **C** | **C** |
| Dehymuls^{®} PGPH | 3 | 1 | 5 | 1 | 1 | 3 | 2 | 4 | 0.5 | 1 | 4 |
| Monomuls^{®} 90-O18 | | 1 | | | | | | | | | |
| Lameform^{®} TGI | | | | | 4 | | | 1 | | 3 | 1 |
| Abil^{®} EM 90 | | | | 1 | | | | | | 2 | |
| Glucate^{®} DO | | | | 3 | | | | | 2 | | |
| Isolan^{®} PDI | | 3 | | | | | 4 | | | | |
| Arlacel^{®} 83 | | | | | | 3 | | | | | |
| Elfacos^{®} ST9 | | | | | | | | | | | 2 |
| Elfacos^{®} ST37 | 2 | | | | | | | | | | |
| Arlacel^{®} P 135 | | | | | | 3 | | | | | |
| Dehymuls^{®} HRE 7 | | | | | | | | | 4 | | |
| Zinkstearat | | 2 | 2 | 1 | 1 | | | 1 | 1 | | |
| Bienenwachs | | 1 | | 2 | 2 | | 3 | | 2 | 1 | 1 |
| Tego^{®} Care CG | | | | | | | | | | | |
| Isostearinsäure | 1 | 1 | | | | | 1 | 1 | | 1 | 1 |
| Wollwachsalkohol, wasserfrei USP | | | | | | | | | | | |
| Antaron V 220 | | 0.5 | 2 | 1 | 1 | 1 | | | | | |
| Kohlenwasserstoff-Gemisch nach Herstellbsp. 1 od. 2 | 2 | 4 | 3 | 3 | 2 | 2 | 1 | 3 | 3 | 1 | 4 |
| Myritol^{®} PC | | | | | | | | | | | |
| Myritol^{®} 331 | 4 | 2 | 3 | | 5 | | | 8 | 5 | 4 | |
| Finsolv^{®} TN | | 5 | 5 | | | 7 | | | | | |
| Cetiol^{®} CC | 3 | 1 | | | | | 3 | 16 | | | 12 |
| Cetiol^{®} OE | | 3 | | 2 | | | 3 | | | | |
| Dow Corning DC^{®} 244 | | 4 | | 2 | | | | | | | |
| Dow Corning DC^{®} 2502 | | | | 1 | | | | | | | |
| Prisorine^{®} 3578 | | 1 | | | | | | | | | |
| Silikonöl Wacker AK^{®} 350 | | | | 1 | | | | | | | |
| Cetiol^{®} 868 | | | | | | | | | | | |
| Eutanol^{®} G 16 | | | | | | | | | | | 3 |
| Eutanol^{®} G 16S | | | | | | | | | | | 7 |
| Cetiol^{®} J 600 | | | | 3 | | | | | | | |
| Ceraphyl^{®} 45 | | | | 1 | | | | | 5 | 4 | |
| Cetiol^{®} B | | | | | 3 | 3 | | 2 | 2 | | |
| Eutanol^{®} G | | | | 2 | | | 4 | | 5 | | |
| Cetiol^{®} PGL | | | | | | | 5 | 2 | | | |
| Mandelöl | | | 2 | | | | | | | | |
| Photonyl^{®} LS | | | | | | | 3 | | | | 2 |
| Panthenol | 1.0 | | | | | | | | | | |
| Bisabolol | 0.2 | | | | | | | | | | |
| Tocopherol / Tocopherylacetat | 1.0 | | | | | | | | | | |
| Magnesiumsulfat x 7 Wasser | 1 | | | | | | | | | | |
| Neo Heliopan^{®} Hydro (Na-Salz) | | 4 | | | | | | 4 | | | |
| Neo Heliopan AP (Na-Salz) | 2 | | | 1 | 2 | | 1 | | | | |
| Neo Heliopan^{®} 303 | 6 | 2 | | | | | | | 6 | | |
| Neo Heliopan^{®} BB | | 2 | | 2 | | 2 | | | | | |
| Neo Heliopan^{®} MBC | 2 | | | | 3 | | 4 | | 2 | | |
| Neo Heliopan^{®} OS | | | | | 10 | | 8 | | | | |
| Neo Heliopan^{®} E 1000 | | | 5 | 6 | | | | | | 5 | |
| Neo Heliopan^{®} AV | | 5 | 5 | | | 7.5 | | | | 5 | |
| Uvinul^{®} T 150 | 1 | | | 2 | 2 | | | | 3 | 2 | |
| Parsol^{®} 1789 | | 1 | 1 | | | | 1 | | 0.5 | | |
| Z-Cote^{®} HP 1 | 4 | 10 | | | | | | 5 | | | 5 |
| Titandioxid T 805 | | | | 2 | | 3 | | 7 | | 4 | 7 |
| Ethanol | | | | 8 | | 10 | | | | | |
| Butylenglykol | 5 | 1 | | 3 | 3 | | | | 8 | 2 | |
| Glycerin | | | 6 | 2 | | | 5 | 5 | | 3 | 5 |
| Wasser, Konservierungsmittel | ad 100, q.s. | | | | | | | | | | |

**Tabelle 5: W/O-Pflegeemulsionen**

| **Komponente** | **45** | **46** | **47** | **48** | **49** | **50** | **51** | **52** | **53** | **54** | **55** |
|---|---|---|---|---|---|---|---|---|---|---|---|
| **L = Lotion. C = Creme** | **C** | **L** | **C** | **L** | **C** | **L** | **L** | **L** | **C** | **C** | **C** |
| Dehymuls^{®} PGPH | 1 | 3 | 1 | 2 | 3 | 1 | 1 | 2 | 1 | 1 | 1 |
| Monomuls^{®} 90-O18 | 2 | | | | | | | | 2 | | 2 |
| Lameform^{®} TGI | 4 | 1 | | | 3 | | | 1 | 4 | 3 | 3 |
| Abil^{®} EM 90 | | | | | | | 4 | | | | |
| Isolan^{®} PDI | | | | | | 4 | | | | | |
| Glucate^{®} DO | | | | 5 | | | | | | | |
| Arlacel^{®} 83 | | | 5 | | | | | | | | |
| Dehymuls^{®} FCE | | | | | | | | | | | |
| Dehymuls^{®} HRE 7 | | | | | | | | 4 | | 1 | |
| Zinkstearat | 2 | 1 | | 1 | 1 | | | 1 | 1 | 1 | |
| Bienenwachs | 2 | | 3 | 1 | | 1 | | 1 | 3 | 2 | 1 |
| Tego Care^{®} CG | | | | | 1 | | | | | | 0.5 |
| Prisorine^{®} 3505 | | | 1 | 1 | | 1 | 1 | | | | 1 |
| Dry Flo^{®} Plus | | | | | | | | | | | |
| SFE 839 | | | | | | | 3 | | | | |
| Lanolin; anhydrous USP | | | 5 | | | | | | | 4 | |
| Kohlenwasserstoff-Gemisch nach Herstellbsp. 1 od. 2 | 3 | 4 | 2 | 12 | 10 | 2 | 2 | 6 | 3 | 12 | 1 |
| Cegesoft^{®} C 17 | | | 3 | | | | | | | 1 | |
| Myritol^{®} PC | | | | | | 2 | | 4 | | | |
| Myritol^{®} 331 | 6 | | | | 2 | 6 | 2 | | | | |
| Finsolv^{®} TN | | | | 5 | | 2 | 5 | | | | |
| Cetiol^{®} A | | 6 | | | | 4 | | | | | |
| Cetiol^{®} CC | | 8 | | | 2 | 2 | 2 | | | | 5 |
| Cetiol^{®} SN | | 5 | | | | | | 3 | | | |
| Cetiol^{®} OE | 3 | | | | 4 | | 2 | | 4 | 2 | 8 |
| Dow Corning DC^{®} 244 | | | | | 1 | | 2 | | | | |
| Dow Corning DC^{®} 2502 | | | 1 | | 2 | | | | | | |
| Prisorine^{®} 3758 | | | | | 3 | | | | | | |
| Silikonöl Wacker AK^{®} 350 | | | | 4 | | | | 3 | | | |
| Cetiol^{®} 868 | | | | | | | | | | 2 | |
| Cetiol^{®} J 600 | | | 4 | | | 2 | | | | | |
| Ceraphyl^{®} 45 | | | | 2 | | | | 2 | | 6 | |
| Cetiol^{®} B | | | 2 | 4 | | | | | | 3 | |
| Eutanol^{®} G 16 | | 1 | | | | | | | | 3 | 7 |
| Eutanol^{®} G | | | 3 | | 1 | | | 8 | | | |
| Cetiol^{®} PGL | | | | | | 4 | | | 9 | | |
| Mandelöl | | | | | 3 | | 5 | | | | |
| Insect Repellent^{®} 3535 | 2 | | | | | | | | | | |
| N,N-Diethyl-m-toluamid | | | | 3 | | | | 5 | | | |
| Photonyl^{®} LS | 2 | 2 | | | | | | | | | |
| Panthenol | 1.0 | | | | | | | | | | |
| Bisabolol | 0.2 | | | | | | | | | | |
| Tocopherol / Tocopheryl Acetate | 1.0 | | | | | | | | | | |
| Magnesiumsulfat x 7 Wasser | 1 | | | | | | | | | | |
| Bentone^{®} 38 | | | | | 1 | | | | | | |
| Aluminum chlorohydrate | | 8 | | | | | | | | | |
| Propylencarbonat | | | | | 0.5 | | | | | | |
| Ethanol | | | | | | | | | | 8 | |
| Butylene Glycol | | | 2 | 6 | | | 2 | 5 | | | 2 |
| Glycerin | 5 | 3 | 3 | | 5 | 3 | 2 | | 10 | 4 | |
| Wasser, Konservierungsmittel | ad 100, q.s. | | | | | | | | | | |

**Tabelle 6: W/O-Pflegeemulsionen**

| **Komponente** | **56** | **57** | **58** | **59** | **60** | **61** | **62** | **63** | **64** | **65** | **66** |
|---|---|---|---|---|---|---|---|---|---|---|---|
| **L = Lotion. C = Creme** | **L** | **C** | **L** | **L** | **C** | **L** | **L** | **L** | **L** | **C** | **C** |
| Dehymuls^{®} PGPH | 3 | 1 | 5 | 1 | 1 | 3 | 3 | 4 | 1 | 1 | 1 |
| Monomuls^{®} 90-O18 | | 1 | | 1 | | | | | | | |
| Lameform^{®} TGI | | | | | 4 | | | 1 | | 3 | |
| Abil^{®} EM 90 | | | | 3 | | | | | | 2 | |
| Isolan^{®} PDl | | 3 | | | | | | | | | 4 |
| Glucate^{®} DO | 1 | | | | | | | | | | |
| Arlacel^{®} 83 | | | | | | 3 | | | | | |
| Dehymuls^{®} FCE | | | | | 4 | | 1 | | | | |
| Dehymuls^{®} HRE 7 | | | | | | | | | 7 | | |
| Zinkstearat | | 2 | 2 | 1 | 1 | 1 | | 1 | 1 | | 1 |
| Bienenwachs | | 4 | | 2 | 1 | 2 | 2 | 1 | 2 | 3 | 5 |
| Tego^{®} Care CG | | | | | | | | | | | |
| Prisorine^{®} 3505 | 1 | 1 | | | | | 1 | 1 | | 1 | 1 |
| Dry Flo^{®} Plus | 1 | | | | | | | | | | |
| SFE^{®} 839 | | 5 | | | | 4 | | | | | |
| Emery^{®} 1780 | | | | | | | | | | | |
| Lanolin anhydrous USP | | 7 | 3 | | | | | | | | |
| Kohlenwasserstoff-Gemisch nach Herstellbsp. 1 od. 2 | 3 | 4 | 4 | 8 | 10 | 2 | 8 | 6 | 3 | 12 | 7 |
| Cegesoft^{®} C 17 | | 2 | | | | | | | | | |
| Myritol^{®} PC | | | | 8 | | | | | | | |
| Myritol^{®} 331 | 4 | | 3 | | 5 | 3 | | | 5 | 4 | |
| Finsolv^{®} TN | | | 5 | | | 7 | | | | | |
| Cetiol^{®} A | | | | | | | | 6 | | | |
| Cetiol^{®} CC | 3 | | | 6 | | 3 | 3 | | | 8 | |
| Cetiol^{®} SN | | | | | 5 | | | | | | |
| Cetiol^{®} OE | | 3 | | 2 | | | 3 | | | | 8 |
| Dow Coming^{®} DC 244 | | 4 | | 2 | | 2 | | | | | |
| Dow Coming^{®} DC 2502 | | | | 1 | | | | | | | |
| Prisorine^{®} 3758 | | | | | | 1 | | | | | |
| Silikonöl Wacker AK^{®} 350 | | | | 1 | | 1 | | 4 | | | |
| Cetiol^{®} 868 | | | | | | | | | | | 10 |
| Cetiol® J 600 | 4 | | | 3 | | | | | | | |
| Ceraphyl® 45 | | | | 1 | | | | | 5 | 4 | |
| Cetiol® B | | | | | 3 | 3 | | 2 | 2 | | |
| Eutanol^{®} G 16 | 1 | | | | | | 3 | | | | |
| Eutanol® G | | | | 2 | | | 2 | | 5 | | |
| Cetiol® PGL | | | 10 | | | | 4 | 6 | | | 3 |
| Mandelöl | | | 2 | | 5 | | 2 | | | | |
| Photonyl^{®} LS | | | | 2 | | | | | | | 2 |
| Panthenol | 1.0 | | | | | | | | | | |
| Bisabolol | 0.2 | | | | | | | | | | |
| Tocopherol / Tocopherylacetat | 1.0 | | | | | | | | | | |
| Magnesiumsulfat x 7 Wasser | 1 | | | | | | | | | | |
| Bentone® 38 | | | | | | 1 | | | | | |
| Propylencarbonat | | | | | | 0.5 | | | | | |
| Ethanol | | | | 8 | | 10 | | | | | |
| Butylenglykol | 5 | 1 | | 3 | 3 | | | | 8 | 2 | 1 |
| Glycerin | | | 6 | 2 | | | 5 | 5 | | 3 | 5 |
| Wasser, Konservierungsmittel | ad 100, q.s. | | | | | | | | | | |

**Tabelle 7: O/W-Pflegeemulsionen**

| **Komponente** | **67** | **68** | **69** | **70** | **71** | **72** | **73** | **74** | **75** | **76** | **77** |
|---|---|---|---|---|---|---|---|---|---|---|---|
| **L = Lotion, C = Creme** | **C** | **C** | **C** | **L** | **C** | **L** | **L** | **C** | **L** | **C** | **C** |
| Eumulgin^{®} VL 75 | | | | | | 4 | | | | | |
| Dehymuls^{®} PGPH | | 2 | | | | | | | | | |
| Generol^{®} R | | | 1 | | | | | | | | |
| Eumulgin^{®} B2 | | | 0.8 | | | | | | | | |
| Tween^{®} 60 | | | | 1 | | | | | | | |
| Cutina^{®} E 24 | | | 0.6 | 2 | | | | | | | |
| Hostaphat^{®} KL 340 N | | | | | | | | | 2 | | |
| Lanette^{®} E | | | | | | | | 1 | | | |
| Amphisol^{®} K | | 0.5 | | | | 1 | | | | 1 | 0.5 |
| Natriumstearat | | | | | 0.5 | | | | | | |
| Emulgade^{®} PL 68/50 | | 2.5 | | | | | | | | 4 | |
| Tego^{®} Care CG | | | | | | | | | | | 2 |
| Tego^{®} Care 450 | | | | | | | | 5 | | | |
| Cutina^{®} MD | | 1 | | 6 | 5 | | 4 | | | 6 | |
| Lanette^{®} 14 | | | | 1 | | | | 2 | | | 4 |
| Lanette^{®} O | 4.5 | | 4 | | 1 | 2 | | | | | 2 |
| Novata^{®}AB | | 1 | | | | | | | | | 1 |
| Lanolin, wasserfrei, USP | | | | | | | 5 | | | | |
| Cetiol^{®} SB 45 | | | 1.5 | | | | 2 | | | | |
| Kohlenwasserstoff-Gemisch nach Herstellbsp. 1 od. 2 | 3 | 4 | 2 | 1 | 10 | 2 | 2 | 6 | 3 | 12 | 1 |
| Cegesoft^{®} C 17 | | | | | | | | | | | |
| Myritol^{®} PC | | | | | 5 | | | | | | |
| Myritol^{®} 331 | 2 | 5 | | | | 6 | | 12 | | | |
| Finsolv^{®} TN | | | | | | 2 | | | 8 | | |
| Cetiol^{®} CC | 4 | 6 | | | | 4 | 4 | | | | 5 |
| Cetiol^{®} OE | | | 7 | | | | | | 4 | 3 | |
| Dow Coming DC^{®} 245 | | | 2 | | 5 | 1 | | | | | |
| Dow Coming DC^{®} 2502 | | | | | 2 | 1 | | | | | |
| Prisorine^{®} 3758 | | | | | | 1 | | | | | |
| Silikonöl Wacker AK^{®} 350 | 0.5 | 0.5 | 0.5 | | | 1 | 4 | | | | |
| Cetiol^{®} 868 | | | | | 2 | | 4 | | | | |
| Cetiol^{®} J 600 | 2 | | 3 | | 3 | 2 | | | | 5 | |
| Ceraphyl® 45 | | | | | | | 3 | | | | |
| Cetiol® SN | | | 5 | | | | | | | | |
| Cetiol® B | | | | | | | | | | 2 | |
| Eutanol® G | | 2 | | 5 | | | | | | | |
| Cetiol® PGL | | | | 7 | | | | | 5 | 5 | |
| Dry Flo® Plus | 5 | | | | | | 1 | | | | |
| SFE 839 | 5 | | | | | | | | | | 2 |
| Mandelöl | | | | | | | 1 | | | | |
| Insect Repellent^{®} 3535 | | 2 | 4 | | | 2 | | | | 3 | |
| N,N-Diethyl-m-toluamid | | 2 | | | | | | | | 3 | |
| Photonyl^{®} LS | 2 | 2 | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum® ultra | | | | | | | | | 1 | | |
| Keltrol® T | | | 0.4 | | | | | | 0.5 | | |
| Pemulen® TR 2 | 0.3 | | | | | | | 0.3 | | | |
| Carbopol® Ultrez 10 | | | 0.3 | 0.2 | | 0.2 | | | 0.1 | 0.3 | |
| Cosmedia SP | | 0,3 | | | 0.2 | | | | | | 0.2 |
| Aluminum Chlorohydrate | | | 7 | | | | | | | | |
| Ethanol | | | | | | | | | | 10 | |
| Butylenglykol | | | | 4 | 3 | | 2 | 5 | 2 | | |
| Glycerin | 2 | 5 | 5 | | 3 | 3 | 2 | | 4 | | 3 |
| Wasser, Konservierungsmittel, NaOH | ad 100, q.s., | | | | | | | | | | |
| | pH 6,5 - 7,5 | | | | | | | | | | |

**Tabelle 8: O/W-Pflegeemulsionen**

| **Komponente** | **78** | **7**9 | **80** | **81** | **82** | **83** | **84** | **85** | **86** | **87** | **88** |
|---|---|---|---|---|---|---|---|---|---|---|---|
| **L = Lotion, C= Creme** | **C** | **C** | **L** | **C** | **L** | **C** | **L** | **L** | **L** | **L** | **C** |
| Eumulgin^{®} VL 75 | 4 | 3 | | | | | 1 | | | | 2 |
| Generol® R | | | | | | 2 | | | | | |
| Eumulgin® B2 | | | | | | 2 | | | | 1 | |
| Tween® 60 | | | | | | | | | | 1 | |
| Cutina® E24 | | | | 2 | | | | | | | |
| Hostaphat^{®} KL 340 N | | | | | | | | | | | |
| Lanette® E | 0.5 | | | | | | | | | | 1 |
| Amphisol® K | 0.5 | 1 | | | | | | 1 | 1 | | |
| Natriumstearat | | | | | 1 | | | | | | |
| Emulgade® PL 68/50 | | 6 | | | | | | 5 | | | 4 |
| Tego® Care CG | | | | | | | | | | | |
| Tego® Care 450 | | | | | | | | | 4 | | |
| Cutina® MD | 3 | | 3 | 8 | 6 | 8 | | | | 4 | |
| Lanette® 14 | | 2 | | | | | | 2 | | 1 | |
| Lanette® O | 2 | | | 2 | | 3 | 1 | | 1 | 1 | 6 |
| Novata® AB | | | | | | | | | | | |
| Lanolin, wasserfrei, USP | | | | | | 4 | | | | | |
| Cetiol® SB 45 | | | | | | | 2 | | | | |
| Kohlenwasserstoff-Gemisch nach Herstellbsp. 1 od. 2 | 3 | 4 | 2 | 1 | 10 | 2 | 2 | 6 | 3 | 12 | 1 |
| Cegesoft® C 17 | 4 | | | | | | | | | | |
| Myritol® PC | 6 | | | | | 5 | | | 5 | | |
| Myritol® 331 | 5 | | 5 | | | | 7 | | | 10 | 3 |
| Finsolv® TN | | 5 | | | 5 | | | 3 | 3 | | 1 |
| Cetiol® CC | | | | | | | | | | | 2 |
| Cetiol® OE | | | | | 2 | | 2 | | 5 | | |
| Dow Corning DC® 245 | | 2 | | | 1 | | | | | 8 | 2 |
| Dow Coming DC® 2502 | | 1 | | | 1 | | | | | | 3 |
| Prisorine^{®} 3758 | 3 | | | | | | | | | | 2 |
| Silikonöl Wacker AK^{®} 350 | | | | | 1 | | | | | | 1 |
| Cetiol^{®} 868 | | 2 | | | | | | | | | |
| Cetiol^{®} J 600 | | 2 | | | | | | | | | |
| Ceraphyl^{®} 45 | | | | | | | 3 | | | | |
| Cetiol^{®} SN | | | | | | | | | | | |
| Cetiol^{®} | | | 5 | | | 5 | | 4 | | | 3 |
| Eutanol^{®} G | | 3 | 5 | | 5 | | | | | | |
| Cetiol^{®} PGL | | | | | | | | 5 | 2 | | |
| Dry Flo^{®} Plus | | 1 | | | | | | | | | 1 |
| SFE 839 | 1 | 1 | | | | | | | | | |
| Mandelöl | | | | | | 2 | | | | | |
| Photonyl^{®} LS | | | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum^{®} Ultra | | | | | | | | | 1 | | |
| Keltrol^{®} T | | | | | | | | | 0.5 | | |
| Carbopol^{®} ETD 2001 | | 0.3 | | 0.3 | | 0.5 | 0.2 | 0.2 | | | |
| Pemulen^{®} TR 2 | | | 0.3 | | | 0.3 | | | | | 0.5 |
| Ethanol | | 5 | | 8 | | | | | | | 10 |
| Butylenglykol | 5 | | 2 | 3 | 3 | | | | | 8 | |
| Glycerin | 2 | 4 | 3 | 3 | | 7 | 5 | 3 | 5 | | |
| Wasser, Konservierungsmittel, NaOH | ad 100, q.s. | | | | | | | | | | |
| | (pH 6,5 - 7,5) | | | | | | | | | | |

**Tabelle 9: Sprayformulierungen**

| **Komponente** | **89** | **90** | **91** | **92** | **93** | **94** | **95** | **96** | **97** | **98** | **99** |
|---|---|---|---|---|---|---|---|---|---|---|---|
| **S = Körperspray, S* = Sonnenschutzspray** | **S** | **S** | **S** | **S** | **S** | **S*** | **S*** | **S*** | **S*** | **S*** | **S*** |
| Emulgade^{®} SE-PF | 8,9 | | 7,5 | 7.5 | 4,3 | 9.8 | 8.2 | 9.9 | | | |
| Eumulgin^{®} B2 | 3,1 | | 3 | | | | | 4.2 | | | |
| Eumulgin^{®} B3 | | | | | | 4.2 | 3.3 | | | | |
| Eumulgin^{®} HRE 40 | | | | | 4,7 | | | | | | |
| Cutina^{®} E 24 | | 5,9 | | 4 | | | | | | | |
| Amphisol^{®} K | | | | | | | | | 1 | 1 | 1 |
| Eumulgin^{®} VL 75 | | | | | | | | | | | 2 |
| Emulgade^{®} PL 68/50 | | 0.5 | | | | | | | 2.5 | 1 | |
| Cutina^{®} MD | | 3,1 | | | | | | | | | |
| *Antaron V 220* | | | | | | 1 | 1 | 1 | | 1 | 1 |
| Kohlenwasserstoff-Gemisch gemäß Herstellbsp. 1 od. 2 | 11 | 5 | 7 | 7 | 7 | 5 | 4 | 5 | 5 | 4 | 6 |
| Myritol^{®} PC | | | | | | | | | | | |
| Myritol^{®} 331 | | | 3 | 4 | 3 | 3 | 3 | 3 | | | |
| Finsolv^{®} TN | | 4 | | | | | | 8 | | | |
| Cetiol^{®} CC | 6 | | | 5 | 5 | 2 | 2 | 4 | | | |
| Cetiol^{®} OE | | 5 | 7 | | | 2 | | | | | |
| Dow Corning DC^{®} 244 | | 4 | 4 | 5 | | | | | | | |
| Cetiol^{®} 868 | 3 | | | | | | | | | | |
| Cetiol^{®} J 600 | | | | 2 | 2 | | | | | | |
| Cetiol^{®} B | | | | | | | 2 | | | | |
| Eutanol^{®} G | 2 | | | | 1 | | | | | | |
| Photonyl^{®} LS | 2 | | | | | | 2 | | | 2 | 2 |
| Panthenol | 1 | | | | | | | | | | |
| Bisabolol | 0,2 | | | | | | | | | | |
| Tocopherol / Tocopherylacetat | 1 | | | | | | | | | | |
| Neo Heliopan^{®} Hydro (Na-Salz) | | | | | | 2 | | | | 3 | |
| Neo Heliopan AP (Na-Salz) | | | | | | 2 | 2 | 2 | | | 1 |
| Eusolex^{®} OCR | | | | | | | 2 | | | | 3 |
| Neo Heliopan^{®} BB | | | | | | | | | | 1 | |
| Neo Heliopan^{®} MBC | | | | | | 2 | 2 | 2 | | 1 | 1 |
| Neo Heliopan^{®} OS | | | | | | 5 | | | | | |
| Neo Heliopan^{®} AV | | | | | | 6 | 6 | 2 | | 7.5 | 2 |
| Uvinul^{®} T 150 | | | | | | 1 | 1 | 1 | | 1 | |
| Parsol^{®} 1789 | | | | | | 1 | | 1 | | 1 | |
| Z-Cote^{®} HP 1 | | | | | | | | | | 2 | 2 |
| Eusolex^{®} T 2000 | | | | | | | | | | 2 | 2 |
| Veegum^{®} Ultra | | | | | | | | | | | 1.5 |
| Laponite^{®} XLG | | | | | | | | | | 1.5 | |
| Keltrol^{®} T | | | | | | | | | | | 0.5 |
| Pemulen^{®} TR 2 | | | | | | | | | 0.2 | | |
| Insect Repellent^{®} 3535 | 1 | | | | | | | | | | |
| N,N-Diethyl-m-toluamid | 1 | | | | | | | | | | |
| Ethanol | | | | | | | | | | | |
| Butylenglykol | | | | | | | 1 | | | 2 | 1 |
| Glycerin | | | | | | 3 | 2 | 3 | 2 | | 3 |
| Wasser/ Konservierungsmittel/NaOH | ad 100/q.s./q.s | | | | | | | | | | |

**Tabelle 10: Antiperspirant Suspensionsstifte sowie Soft Solids - Mengenangaben in Gew.-% -**

| **Zusammansetzung** | **1** | **2** | **3** | **4** | **5** | **6** |
|---|---|---|---|---|---|---|
| Distearylether | - | 15 | - | - | - | - |
| Stearylalkohol | - | - | 15 | 10 | 14,7 | - |
| Guerbetalkohol C36 | 15 | - | - | - | - | - |
| Tribehenin | - | - | - | - | - | 20 |
| Hydrogenated Castor Oil | - | - | 4 | - | 3,7 | - |
| Erfindungsgemäßes Kohlenwasserstoff Gemisch nach Herstellbeispiel 1 od. 2 | 30 | 60 | 51 | 60 | 31,6 | 55 |
| Octyldodecanol | 5 | - | - | - | - | - |
| Dicaprylylether | 5 | - | - | - | - | - |
| Hexyldecanol + Hexyldecyl Laurate | - | 10 | - | - | - | - |
| Cyclomethicone | 20 | - | - | - | 30 | - |
| Dry Flo Plus* | - | - | 5 | - | - | - |
| Silica | - | - | - | 2,5 | - | - |
| Talc | - | - | - | 2,5 | - | - |
| Aluminium Zirconium Tetrachlorohydrex GLY | - | - | 25 | 25 | - | 25 |
| Aluminium Chlorohydrate | 25 | 15 | - | - | 20 | - |

| | | | | | | |
|---|---|---|---|---|---|---|
| * National Starch | | | | | | |

***Forts.: Tabelle 10: Antiperspirant Suspensionsstifte sowie Soft Solids - Mengenangaben in Gew.-% -***

| **Zusammensetzung** | **7** | **8** |
|---|---|---|
| 12-Hydroxystearinsäure | 10 | 5 |
| Erfindungsgemäßes Kohlenwasserstoff Gemisch nach Herstellbsp. 1 od. 2 | 65 | 65 |
| Dry Flo Plus* | - | 5 |
| Aluminium Zirconium Tetrachlorohydrex GLY | 25 | 25 |

| | | |
|---|---|---|
| * National Starch | | |

**Tabelle 11: Rezepturen 1 bis 13**

| **Komponenten INCl (Handelsname)** | **1** | **2** | **3** | **4** | **5** | **6** | **7** | **8** | **9** | **10** | **11** | **12** | **13** |
|---|---|---|---|---|---|---|---|---|---|---|---|---|---|
| Glyceryl Stearate, Ceteareth-20, Ceteareth-12, Cetearyl Alcohol, Cetyl Palmitate (Emulgade^{®} SE) | | | | | | | | | | | | 10.7 | 5.1 |
| Ceteareth-20 (Eumulgin^{®} B2) | | | | | | | | | | | | 5.8 | 3.4 |
| Cetearyl Glucoside, Cetearyl Alcohol (Emulgade^{®} PL 68/50) | 1 | | 1 | 1 | | 2 | 2 | | 2 | | 2 | | |
| Polyglyceryl-2 Dipolyhydroxystearate, Lauryl Glucoside, Glycerin (Eumulgin^{®} VL 75) | | 1 | | | 1 | | | 3 | | 2.5 | | | |
| Sodium Cetearyl Sulfate (Lanette^{®} E) | 1 | 1 | 1 | | 1 | | | | 1 | 1 | | | |
| Kohlenwasserstoff Gemisch nach Herstellbsp. 1 od. 2 | 5 | 4 | 8 | 3 | 5 | 8 | 4 | 2 | 4 | 3 | 5 | 10 | 2 |
| Dicaprylyl Carbonate (Cetiol^{®} CC) | 5 | 5 | 5 | | | | | 4 | | 5 | 3 | 4 | |
| Cocoglycerides (Myritol^{®} 331) | 3 | 4 | | 4 | 4 | | | | 5 | | | 3 | 3 |
| Dicaprylyl Ether (Cetiol^{®} OE) | | | | | 5 | | 3 | | 2 | | | | |
| Dibutyl Adipate (Cetiol^{®} B) | | | | 4 | | | | 4 | | 4 | | | |
| Dimer Distearyltricarbonate (Cosmedia^{®} DC) | 1 | 1 | 1 | 1 | 1 | 1.5 | 1.5 | 2 | 3 | 2 | 1.5 | 2 | 2 |
| Ethyl Butylacetylaminopropionate | | | | | | | | | | | 5 | 5 | |
| Tocopherol | 1 | 1 | 1 | 1 | 1 | 1 | 1 | 1 | 1 | 1 | 1 | 1 | 1 |
| Zinc oxide, nanonisiert, gecoated | 5 | 5 | 5 | 5 | 5 | | 2 | | 5 | 3 | | | |
| Titanium dioxide, nanonisiert, Gecoated | | | | 5 | 5 | | 2 | 3 | 5 | 2 | | | |
| Octyl Methoxycinnamate | 7.5 | 7.5 | 7.5 | | | | 3 | 1 | 3 | 5 | | 5 | 5 |
| Octocrylene | 9 | 9 | 9 | | | 2 | 1 | | | | 2 | | 1.5 |
| Butyl Methoxydibenzoylmethane | | | | | | 2 | 2 | | | 1 | 2 | 2 | |
| 4-Methylbenzylidene Camphor | | | | | | | 2 | | | | | | 2 |
| Ethylhexyl Triazone | | | | | | 1 | 1 | 2 | | | 1 | | |
| Diethylhexyl Butamido Triazone | | | | | | 1 | 1 | 2 | | | 1 | 2 | |
| Phenylbenzimidazole Sulfonic Acid als Na-Salz, 15% wäßrige Lsg. | | | | | | | | | | | | | 13.3 |
| Glycerin | 3 | 3 | 3 | 3 | 3 | 3 | 3 | 3 | 3 | 3 | 3 | 5 | 5 |
| Magnesium aluminium silicate (and) cellulose Gum | 0.75 | 0.75 | | 0.5 | 0.5 | | 0.5 | | | 0.35 | | | |
| Xanthan Gum | 0.25 | 0.25 | | 0.5 | 0.5 | | 0.5 | | | 0.35 | | | |
| Sodium Polyacrylate (Cosmedia^{®} SP) | | | 0.1 | | | 0.1 | 0.2 | | | | 0.1 | | |
| Acrylates/C10-30 Alkyl Acrylate Crosspolymer | | | | | | | | 0.2 | 0.1 | | | | |
| Wasser, Perfume, Preservatives | q.s. | q.s. | q.s. | q.s. | q.s. | q.s. | q.s. | q.s. | q.s. | q.s. | q.s. | q.s. | q.s. |

**Tabelle 12: Rezepturen 14 bis 26**

| | | | | | | | | | | | | | |
|---|---|---|---|---|---|---|---|---|---|---|---|---|---|
| | | | | | | | | | | | | | |

| **Komponenten INCl (Handelsname)** | **14** | **15** | **16** | **17** | **18** | **19** | **20** | **21** | **22** | **23** | **24** | **25** | **26** |
|---|---|---|---|---|---|---|---|---|---|---|---|---|---|
| Glyceryl Stearate, Ceteareth-20, Ceteareth-12, Cetearyl Alcohol, Cetyl Palmitate (Emulgade^{®} SE) | 3.7 | 3.7 | | | | | | | | | | 4.9 | 4.1 |
| Ceteareth-12 (Eumulgin^{®}B1) | 1.3 | 1.3 | | | | | | | | | | | |
| Ceteareth-20 (Eumulgin^{®}B2) | | | | | | | | | | | | 1.1 | 0.9 |
| Cetearyl Glucoside, Cetearyl Alcohol (Emulgade^{®} PL 68/50) | | | 5 | 1 | 1 | 1 | 1 | 3 | | | | | |
| Polyglyceryl-2 Dipolyhydroxystearate, Lauryl Glucoside, Glycerin (Eumulgin^{®} VL 75) | | | | | | | | | 3 | 5 | 5 | | |
| Sodium Cetearyl Sulfate (Lanette^{®} E) | | | | 0.25 | 0.25 | 0,25 | 0.25 | 0.25 | | | | | |
| Potassium Cetyl Phosphate | | | 0.5 | | | | | | | | | | |
| Kohlenwasserstoff Gemisch nach Herstellbsp.1 od. 2 | 4 | 5 | 6 | 8 | 5 | 8 | 8 | 10 | 7 | 4 | 10 | 5 | 5 |
| Dicaprylyl Carbonate (Cetiol^{®} CC) | 5 | | 5 | | | | | | 2.5 | 4 | 4 | 5 | 5 |
| Coco-CaprylatelCaprate (Cetiol^{®} LC) | | | | 1 | 1 | 1 | 1 | 1 | | | | | |
| Caprylic/Capric Triglyceride (Myritol^{®} 312) | | | | 1 | 1 | 1 | 1 | 1 | | | | | |
| Cocoglycerides (Myritol^{®} 331) | | | | | | | | | | 4 | 4 | | |
| Cetearyl Isononanoate (Cetiol^{®}SN) | 3 | 3 | 3.5 | | | | | | | | | | |
| Octyldodecanol (Eutanol^{®} G) | | | | | | | | | 3.5 | 2 | 2 | | |
| Hexyldecanol (Eutanol^{®} G16) | | | | 1 | 1 | 1 | 1 | 1 | | | | | |
| Olus (Cegesoft^{®} PS6) | | 1.5 | 1.5 | | | | | | | | | | |
| Passiflora Incamata (Cegesoft^{®} PFO) | 1.5 | | | | | | | | | | | | |
| Dimethicone | | | | 0.5 | 0.5 | 0.5 | 0.5 | 0.5 | | | | | |
| Dimer Distearyltricarbonate (Cosmedia^{®} DC) | 1 | | 1.5 | | | | | 1.5 | | 2,5 | 2,5 | | 0.5 |
| Triethyl Citrate (Hydagen^{®} C.A.T) | | | | | | | | | | | | 1.5 | |
| Tocopherol | | | | | | | | | 0.5 | 0.5 | 0.5 | | |
| Tocopheryl Acetate | 0.5 | 0.5 | | | | | | | | | | | |
| Ethanol | | | | | | | | | | | 5 | | |
| Aluminum Chlorhydrate (Locron L) | | | | | | | | | | | | | 40 |
| Chitosan (Hydagen^{®} DCMF) | | | | | | | | | | | | 0.1 | |
| Glycolic Acid | | | | | | | | | | | | 0.04 | |
| Glycerin | 2 | 2 | 2 | 3 | 3 | 3 | 3 | 3 | 2 | 2 | 2 | 3 | 2 |
| Potassium Hydroxyde, 20% wäßrige Lsg. | | | | | | 0.3 | 0.2 | 0.1 | 0.4 | 0.3 | 0.5 | | |
| Glycerin, Glyceryl Polyacrylate (Hispagel®50) | | | | | | | | | | 10 | | | |
| Carbomer | | | | | | | 0.1 | | 0.2 | | 0.2 | | |
| Sodium Polyacrylate (Cosmedia® SP) | | | | | 0.15 | | | | | | | | |
| Acrylates/C10-30 Alkyl Acrylate Crosspolymer | | | | | | 0.15 | | 0.05 | | | | | |
| Wasser, Perfume, Preservatives | q.s. | q.s. | q.s. | q.s. | q.s. | q.s. | q.s. | q.s. | q.s. | q.s. | q.s. | q.s. | |

**Tabelle 13: Rezepturen 27 bis 33 (Formulierungen für AP/Deo)**

| **Komponenten INCI (Handelsname)** | **27** | **28** | **29** | **30** | **31** | **32** | **33** |
|---|---|---|---|---|---|---|---|
| Glyceryl Stearate, Ceteareth-20, Ceteareth-12, Cetearyl Alcohol, Cetyl Palmitate (Emulgade^{®} SE) | 6 | | 4,5 | | | 6 | |
| Ceteareth-20 (Eumulgin^{®}B2) | | | 1 | | | | |
| Cetearyl Isononanoate, Glyceryl Stearate, Ceteareth-20, | | | | | | | |
| Ceteareth-12, Cetearyl Alcohol, | | | | | | | |
| Cetyl Palmitate (Emulgade^{®} CM) | | | | | 20 | | |
| Polyglyceryl-3 Diisostearate Lameform TGI | | 3 | | | | | |
| Cocoglycerides (Novata^{®} AB) | | | | | | | 4 |
| Stearyl alcohol (Lanette 18) | | | | 14,7 | | | |
| Hydrogenated Castor Oil (Cutina^{®} HR) | | | | 3,7 | | | 6,5 |
| Polyglyceryl-2 Dipolyhydroxystearate, (Dehymuls^{®} PGPH) | | 1 | | | | | |
| Sodium Cetearyl Sulfate (Lanette^{®} E) | 0,3 | | | | | 0,3 | |
| Behenyl Alcohol (Lanette^{®} 22) | 2 | | | | | 4 | |
| Kohlenwasserstoff Gemisch nach Herstellbsp. 1 od. 2 | 4 | 4 | | 5 | 4 | 4 | 15 |
| Dicaprylyl Carbonate (Cetiol^{®} CC) | | 3 | | | | | |
| Dicaprylylether (Cetiol^{®} OE) | 2 | | | 4 | | 3 | 9 |
| Cocoglycerides (Myritol^{®} 331) | | | | | | | |
| Cyclopentasiloxane | 3 | 5 | | 34 | | 2 | 14 |
| Cyclopentasiloxane and DimethiconeNinyldimethicone | | | | | | | |
| Crosspolymer SFE 839 (GE Bayer) | | 3 | | | | | |
| Dimethicone | 1 | | | | | | |
| Dimer Distearyltricarbonate (Cosmedia^{®} DC) | 1 | 1 | | 1 | 1 | 1 | |
| Triethyl Citrate (Hydagen^{®} C.A.T) | | | 2 | | | | |
| PEG-40 Hydrogenated Castor Oil | | | | | 1 | | |
| Tocopheryl Acetate | | | | 1 | | | |
| Aluminium Zirconium Tetrachlorohydrex GLY (Rezal 36) | 30 | | | 22,9 | | 30 | 25 |
| Aluminum Chlorhydrate (Locron L) | | | 10 | | | | |
| Chitosan (Hydagen^{®} DCMF) | 0,05 | | | | | | |
| Glycolic Acid | 0,02 | | | | | | |
| Glycerin | | 5 | 5 | | | | |
| Propylene Carbonate (Fluka) | | | | | | | 0,5 1 |
| Quaternium-18 Hectorite (Bentone 18) | | | | | | | |
| Talc (Merck) | | | | | | 5 | 5 |
| MgSO4x7H2O | | 1 | | | | | |
| Wasser Phase II | 46,7 | | 35 | | | | |
| Wasser, Perfume, Preservatives | q.s. | 40 | q.s. | q.s. | q.s. | q.s. | q.s. |

| | | | | | | | |
|---|---|---|---|---|---|---|---|
| 27 - Antiperspirant / Deo Creme 28 - Antiperspirant Creme (W/O) 29 - Antiperspirant / Deo Spray 30 - Antiperspirant Stift mit Vitamin E 31 - Deodorant Wipe - Formulierung 32 - Antiperspirant Creme 33 - Antiperspirant Creme «Soft Solid » | | | | | | | |

In der Tabelle 14 werden Sonnenschutzformulierungen vom Typ O/W beschrieben, in der Tabelle 15 werden Pflegeemulsionen beschrieben. Durch den Einsatz der erfindungsgemäßen Kohlenwasserstoff Gemische wird das sensorische Verhalten bei Applikation positiv beeinflusst. Die Mengenangaben beziehen sich jeweils auf Gew.-% der handelsüblichen Substanzen in der Gesamtzusammensetzung.

**Tabelle 14: OfW-Sonnenschutzemulsionen**

| Komponente | **12** | **13** | **14** | **15** | **16** | **17** | **18** | **19** | **20** | **21** | **22** |
|---|---|---|---|---|---|---|---|---|---|---|---|
| L = Lotion, C = Creme, S = Spray | L | C | L | C | L | C | S | C | C | L | L |
| Eumulgin^{®} VL 75 | 2 | | | | 3 | | | | 1 | | |
| Eumulgin^{®} B2 | | | | 2 | | | | | | 1 | |
| Tween^{®} 60 | | | | | | | | | | 1 | |
| Cutina^{®} E 24 | | | | 0.5 | | | | | | | |
| Hostaphat^{®} KL 340 N | | | | | | | | | | | 0.5 |
| Eumulgin^{®} SG | | | 0,5 | | | 0,5 | | 0,3 | 0,1 | | |
| Lanette^{®} E | | | | | | | 0.1 | | 0.5 | | |
| Amphisol^{®} K | 0.5 | | | | | | 1 | | | | |
| Natriumstearat | | | | | 1 | | | | | | |
| Emulgade^{®} PL 68/50 | | 2 | 1 | | | 3 | | | | | |
| Tego^{®} Care 450 | | 2 | | | | | | | 2 | | |
| Cutina^{®} MD | | | | 2 | 1 | 3 | | | | | 1 |
| Lanette^{®}14 | | 1 | | | | | | | | | |
| Lanette^{®} 0 | | | | 2 | | | | 2 | 1 | 1 | |
| Cutina^{®} PES | 1 | 1 | | 2 | | | | | | 1 | |
| Allianz^{®} OPT | 1 | | | 1 | 1 | | | 2 | | | 2 |
| Cosmedia^{®} DC | | 1.5 | 2 | | | 1.5 | 2 | | 1.5 | 1.5 | |
| Lanolin, wasserfrei, USP | | | | | | 1 | 1 | | | | |
| Kohlenwasserstoff Gemisch nach Herstellbsp. 1 od. 2 | 6 | 2 | 4 | 7 | 3 | 7 | 6 | 6 | 4 | 4 | 5 |
| Myritol^{®} PC | | | | | | | | | 5 | | |
| Myritol^{®} 331 | 6 | | 4 | | | 5 | 8 | | | 10 | 8 |
| Finsolv^{®} TN | | | | | 5 | | | 3 | 3 | | |
| Cetiol^{®} CC | 6 | | 6 | | | 5 | 5 | | | | |
| Cetiol^{®} OE | | | | | 2 | | | | | | 2 |
| Dow Coming DC^{®} 244 | | 2 | | | 1 | | | | | | |
| Dow Coming DC^{®} 2502 | | 1 | | | 1 | | | 3 | | | |
| Ceraphyl^{®} 45 | | | | | | | | | | 2 | 2 |
| Silikonöl Wacker AK^{®} 350 | | | | | 1 | | | | | | |
| Cetiol^{®} 868 | | 2 | | | | | | | | | |
| Cetiol^{®} J 600 | | 2 | | | | | | | | | |
| Cetiol^{®} B | 4 | | 4 | | | | | 4 | | | |
| Eutanol^{®} G | | 3 | | | | 3 | | | | | |
| Eutanol^{®} G 16 S | 3 | | | | | | | | | | |
| Cetiol^{®} PGL | | | | | | | | | 2 | | |
| Photonyl^{®} LS | | | | | | | | | | 2 | |
| Panthenol | 1 | | | | | | | | | | |
| Bisabolol | 0,2 | | | | | | | | | | |
| Tocopherol / Tocopherylacetat | 1 | | | | | | | | | | |
| Neo Heliopan^{®} Hydro (Na-Salz) | | | | | | | | | | 3 | |
| Eusolex^{®} OCR | 6 | | 9 | | 5 | 7 | 9 | | 4 | | 7 |
| Neo Heliopan^{®} AP (Na-Salz) | | | | 0.5 | | 1 | | | | | |
| Neo Heliopan^{®} BB | | | | | | | | 1 | 1 | | 1 |
| Neo Heliopan^{®} MBC | | 2 | | 1 | | | | 3 | 1 | | 3 |
| Neo Heliopan^{®} OS | 2 | | | | | | | | 7 | | |
| Neo Heliopan^{®} E1000 | | 4 | | | | | | 5 | | | |
| Neo Heliopan^{®} AV | | 4 | 7.5 | 5 | | | | 5 | 4 | 7.5 | |
| Uvinul^{®} A PLUS | | | | | 1 | | 2 | | | | |
| Uvinul^{®} T 150 | 1 | | | | | | | | 1.3 | 1 | 1 |
| Tinosorb^{®} M | | 2 | | | 2 | | 2 | | | | |
| Tinosorb^{®} S | | 1 | | | 2 | | 2 | | | | |
| Parsol^{®} 1789 | 1 | | | | | | | | 2 | | 1 |
| Z-Cote^{®} HP1 | 7 | 2 | 5 | | | 7 | 5 | | 6 | 2 | |
| Eusolex^{®} T 2000 | 5 | 2 | | | 10 | | | 10 | | 2 | |
| Veegum^{®} Ultra | 1.5 | | 1.5 | | | 1.5 | 1.2 | | 1 | | |
| Keltrol^{®} T | 0.5 | | 0.5 | | | 0.5 | 0.4 | | 0.5 | | |
| Cosmedia^{®} SP | | | 0.2 | 0.3 | | | 0.1 | | | 0.2 | |
| Pemulen^{®} TR2 | | 0.3 | | 0.3 | | | | 0.2 | | | 0.3 |
| Ethanol | | 5 | | 8 | | | | | | | |
| Butylenglykol | 1 | | | 3 | 3 | | | | | 8 | 1 |
| Glycerin | 2 | 4 | 3 | 3 | | 3 | 3 | 3 | 5 | | 3 |
| Wasser/ Konservierungsmittel/ NaOH | ad 1001 q.s./q.s | | | | | | | | | | |

**Tabelle 15: O/W-Pflegeemulsionen**

| **Komponente** | **34** | **35** | **36** | **37** | **38** | **39** | **40** | **41** | **42** | **43** | **44** |
|---|---|---|---|---|---|---|---|---|---|---|---|
| **L = Lotion, C = Creme** | C | C | L | C | L | C | L | L | L | L | C |
| Eumulgin^{®} VL 75 | | | 5 | | 4 | | | | | | 2 |
| Generol^{®} R | | | | | | 2 | | | | | |
| Eumulgin^{®} B2 | | | | | | | | | | 1 | |
| Tween^{®} 60 | | | | | | | | | | 1 | |
| Cutina^{®} E 24 | | | | 0.5 | | | | | | | |
| Eumulgin^{®} SG | | | 0,1 | 0,5 | | 0,4 | | 0,2 | 0,1 | | |
| Lanette^{®} E | 0.5 | | | | | | | | | | |
| Amphisol^{®} K | 0,5 | 0.5 | | | | | | | | | |
| Natriumstearat | | | | | 1 | | | | | | |
| Emulgade^{®} PL 68/50 | | 2 | | 2 | | | | 3 | 4 | | |
| Tego^{®} Care 450 | | 1 | | | | | | | 1 | | |
| Cutina^{®} MD | 2 | 1 | 1 | 1 | | 5 | | | | 2 | |
| Lanette^{®} 14 | | | | | 1 | | | 2 | | 1 | |
| Lanette® 0 | 2 | | | 2 | 1 | 3 | 1 | | 1 | 1 | 3 |
| Cutina^{®} PES | 1 | 2 | | 3 | 1 | | | | | | 3 |
| Novata^{®} AB | | | | | | | | | 1 | 1 | |
| Lanolin, wasserfrei, USP | | | | | | 4 | | | | | |
| Cosmedia^{®} DC | | | 2 | | | 1.5 | | | 1 | 1 | |
| Cetiol^{®} SB 45 | | | | | | | 2 | | | | |
| Cegesoft^{®} C 17 | 2 | | | | | | | | | | |
| Kohlenwasserstoff Gemisch nach Herstellbsp. 1 od. 2 | 5 | 5 | 4 | 4 | 3 | 4 | 5 | 4 | 5 | 10 | 2 |
| Myritol^{®} PC | 6 | | | | | 5 | | | | | |
| Myritol^{®} 331 | 2 | | 5 | | | | 2 | | | | 3 |
| Finsolv^{®} TN | | | | 3 | 5 | | | 3 | 3 | | 1 |
| Cetiol^{®} CC | | | | 3 | | | 4 | 3 | | | |
| Cetiol^{®} OE | | | | | 2 | | 2 | | 5 | | |
| Dow Coming DC^{®} 245 | | 2 | | | 1 | 4 | | | | 8 | 2 |
| Dow Coming DC^{®} 2502 | | 1 | | | 1 | | | | | | 3 |
| Prisorine^{®} 3758 | 3 | | | | | | | | | | 2 |
| Silikonöl Wacker AK^{®} 350 | | | | | 1 | | | | | | 1 |
| Cetiol^{®} 868 | | 2 | | | | | | | | | |
| Cetiol^{®} J 600 | | 2 | | 2 | | | | | | | |
| Ceraphyl^{®} 45 | | | | | | | 3 | | | | |
| Cetiol^{®} SN | | | | 5 | | | | | | | |
| Cetiol^{®} B | | | 5 | | | 5 | | 4 | | | 3 |
| Eutanol^{®} G | | 3 | 5 | | 5 | | | | | | |
| Cetiol^{®} PGL | | | | | | | | 5 | 2 | | |
| Dry Flo^{®} Plus | | 1 | | | | | | | | | 1 |
| SFE 839 | 1 | 1 | | | | | | | | | |
| Mandelöl | | | | | | 2 | | | | | |
| Photonyl^{®} LS | | | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum^{®} Ultra | | | | | | | | | 1 | | |
| Keltrol^{®} T | | | | | | | | | 0.5 | | |
| Cosmedia^{®} SP | 0.5 | | | | | 0.5 | 0.5 | 0.2 | | | 0.5 |
| Carbopol^{®} ETD 2001 | | 0.3 | | 0.3 | | | | | | | |
| Pemulen^{®} TR 2 | | | 0.3 | | | 0.3 | | | | | |
| Ethanol | | 5 | | 8 | | | | | | | 10 |
| Butylenglykol | 5 | | 2 | 3 | 3 | | | | | 8 | |
| Glycerin | 2 | 4 | 3 | 3 | | 7 | 5 | 3 | 5 | | |
| Wasser, Konservierungsmittel, NaOH | ad 100, q.s. | | | | | | | | | | |
| | (pH 6,5 - 7,5) | | | | | | | | | | |

### Formulierungen für den Sonnenschutz und die Hautpflege vom Typ Wasser in Öl

In Tabelle 16 werden Sonnenschutzformulierungen vom W/O Emulsionstyp, in Tabelle 17 werden Pflegeemulsionen beschrieben. Durch den Einsatz der Kohlenwasserstoff Gemische wird das sensorische Verhalten bei Applikation positiv beeinflusst. Die Mengenangaben beziehen sich jeweils auf Gew.-% der handelsüblichen Substanzen in der Gesamtzusammensetzung.

**Tabelle 16: W/O - Sonnenschutzformulierungen**

| **Ingredient** | **1** | **2** | **3** | **4** | **5** | **6** | **7** | **8** | **9** | **10** | **11** |
|---|---|---|---|---|---|---|---|---|---|---|---|
| **L = Lotion; C = Creme** | C | L | C | L | C | L | L | L | L | C | C |
| Dehymuls^{®} PGPH | 4 | 2 | 1 | 3 | 3 | 1 | 1 | 2 | 2 | 4 | 1 |
| Monomuls^{®} 90-018 | | | 2 | | | | | | | | |
| Lameform^{®} TGI | 2 | | 4 | | 3 | | | | | 1 | 3 |
| Abil^{®} EM 90 | | | | | | | 4 | | | | |
| Isolan^{®} PDI | | | | | | 4 | | 2 | | | |
| Zinkstearat | 1 | | | 1 | 1 | | | 1 | | 1 | |
| Bienenwachs | 1 | | 5 | 1 | | | | 5 | | 7 | 5 |
| Tego^{®} Care CG | | | | | 1 | | | | | | 0.5 |
| Prisorine^{®} 3505 | | | 1 | | | 1 | 1 | | | | 1 |
| Cosmedia DC | 3 | 4 | 2 | 1 | 1 | 2 | 2 | 2 | 3 | 1 | 1 |
| Kohlenwasserstoff Gemisch nach Herstellbsp. 1 od. 2 | 5 | 4 | 4 | 3 | 2 | 4 | 3 | 4 | 2 | 3 | 5 |
| Myritol^{®} 331 | 2 | | | | 3 | 6 | | | | | 3 |
| Finsolv^{®} TN | | | | 5 | | | 2 | | | | |
| Cetiol^{®} CC | 5 | | 2 | | 4 | 2 | | | 2 | 3 | 5 |
| Tegosoft DEC | | 4 | | 3 | | | 5 | 5 | | | |
| Cetiol^{®} OE | | | | | 4 | | 5 | | 4 | 2 | |
| Dow Coming^{®} DC 244 | | | 3 | | | | 2 | | 2 | 4 | |
| Dow Coming^{®} DC 2502 | 1 | | 1 | | 2 | 1 | | | | | 1 |
| Silikonöl Wacker AK 350 | | 1 | | 4 | | | | 3 | | | |
| Cetiol^{®} PGL | | 3 | | | | 2 | | | 4 | | |
| Cophero^{®} F 1300 | | | | | | 1 | | | | | |
| Magnesium sulfat x 7 H₂O | | | | | | 1 | | | | | |
| Neo Heliopan^{®} Hydro (Na-Salz) | 2 | | 2.2 | | 3 | 3 | | | 1 | | 2 |
| Neo Heliopan^{®} 303 | | 5 | | | | | | | 4 | | 4 |
| Uvasorb^{®} HEB | 1 | | | 1 | 1 | | | | | | 2 |
| Neo Heliopan^{®} MBC | 2 | | | | | 2 | 2 | 2 | | | |
| Uvinul^{®} A plus | | | | | 2 | | | | 3 | 3 | |
| Neo Heliopan^{®} AP (Na-Salz) | | 2 | 2 | | 1 | | | | 1 | | 6 |
| Neo Heliopan^{®} AV | 3 | | 4 | 6 | 4 | 7.5 | 4 | 5 | | | 1 |
| Uvinul^{®} T 150 | 1 | 1 | | | 2.5 | | | 1 | | | |
| Parsol^{®} 1789 | 2 | 1 | | | | | 2 | | 2 | 2 | |
| Zinkoxid NDM | | | | | | 10 | | 3 | | | 4 |
| Tinosorb^{®} M | | 3 | | 3 | | | | 2 | | 2 | |
| Tinosorb^{®} S | | 3 | | 3 | | | | 2 | | 2 | |
| Eusolex^{®} T Aqua | | | 8 | | | | | 5 | | | |
| Eusolex^{®} T 2000 | | | | | 5 | | 3 | 3 | | | 4 |
| Ethanol | | | | | | | | | | 8 | |
| Glycerin | 5 | 3 | 3 | 3 | 5 | 3 | 2 | 3 | 10 | 4 | 3 |
| Wasser, Konservierungsmittel | ad 100, q.s. | | | | | | | | | | |

**Tabelle 17: W/O-Pflegeemulsionen**

| **Komponente** | **55** | **56** | **57** | **58** | **59** | **60** | **61** | **62** | **63** | **64** | **65** |
|---|---|---|---|---|---|---|---|---|---|---|---|
| **L = Lotion. C = Creme** | C | L | C | L | C | L | L | L | C | C | C |
| Dehymuls^{®} PGPH | 1 | 3 | 1 | 2 | 3 | 1 | 1 | 2 | 1 | 1 | 1 |
| Monomuls^{®} 90-018 | 2 | | | | | | | | 2 | | 2 |
| Lameform^{®} TGI | 4 | 1 | | | 3 | | | 1 | 4 | 3 | 3 |
| Abil^{®} EM 90 | | | | | | | 4 | | | | |
| Isolan^{®} PDI | | | | | | 4 | | | | | |
| Glucate^{®} DO | | | | 5 | | | | | | | |
| Arlacel^{®} 83 | | | 5 | | | | | | | | |
| Dehymuls^{®} FCE | | | | | | | | | | | |
| Dehymuls^{®} HRE 7 | | | | | | | | 4 | | 1 | |
| Zinkstearat | 2 | 1 | | 1 | 1 | | | 1 | 1 | 1 | |
| Bienenwachs | 4 | | | 1 | | | | 1 | 4 | 7 | |
| Tego Care^{®} CG | | | | | 1 | | | | | | 0.5 |
| Prisorine^{®} 3505 | | | 1 | 1 | | 1 | 1 | | | | 1 |
| Dry Flo^{®} Plus | | | | | | | | | | | |
| SFE 839 | | | | | | | 3 | | | | |
| Lanolin; anhydrous USP | | | 5 | | | | | | | 4 | |
| Kohlenwasserstoff Gemisch nach Herstellbsp. 1 od. 2 | 3 | 4 | 2 | 12 | 10 | 2 | 2 | 6 | 3 | 12 | 1 |
| Cegesoft^{®} C 17 | | | 3 | | | | | | | 1 | |
| Myritol^{®} PC | | | | | | 2 | | 4 | | | |
| Myritol^{®} 331 | 6 | | | | 2 | 6 | 2 | | | | 8 |
| Finsolv^{®} TN | | | | 5 | | 2 | 5 | | | | |
| Cetiol^{®} A | | 6 | | | | 4 | | | | | |
| Cetiol^{®} CC | | 8 | | | 2 | 2 | 2 | | | | 5 |
| Cetiol^{®} SN | | 5 | | | | | | 3 | | | |
| Cetiol^{®} OE | 3 | | | | 4 | | 2 | | 4 | 2 | |
| Dow Coming DC^{®} 244 | | | | | 1 | | 2 | | | | |
| Dow Coming DC^{®} 2502 | | | 1 | | 2 | | | | | | |
| Prisorine^{®} 3758 | | | | | 3 | | | | | | |
| Silikonöl Wacker AK^{®} 350 | | | | 4 | | | | 3 | | | |
| Cetiol^{®} 868 | | | | | | | | | | 2 | 7 |
| Cetiol^{®} J 600 | | | 4 | | | 2 | | | | | |
| Ceraphyl^{®} 45 | | | | 2 | | | | 2 | | 6 | |
| Cetiol^{®} B | | | 2 | 4 | | | | | | 3 | |
| Eutanol^{®} G 16 | | 1 | | | | | | | | 3 | |
| Eutanol^{®} G | | | 3 | | | | | 8 | | | |
| Cetiol^{®} PGL | | | | | | 4 | | | 9 | | |
| Mandelöl | | | | | 1 | | 5 | | | | |
| Insect Repellent^{®} 3535 | 2 | | | | | | | | | | |
| N,N-Diethyl-m-toluamid | | | | 3 | | | | 5 | | | |
| Photonyl^{®} LS | 2 | 2 | | | | | | | | | |
| Panthenol | 1.0 | | | | | | | | | | |
| Bisabolol | 0.2 | | | | | | | | | | |
| **Tocopherol / Tocopheryl Acetate** | 1.0 | | | | | | | | | | |
| Magnesiumsulfat x 7 Wasser | 1 | | | | | | | | | | |
| Bentone^{®} 38 | | | | | 1 | | | | | | |
| Propylencarbonat | | | | | 0.5 | | | | | | |
| Ethanol | | | | | | | | | | 8 | |
| Butylene Glycol | | | 2 | 6 | | | 2 | 5 | | | 2 |
| Glycerin | 5 | 3 | 3 | | 5 | 3 | 2 | | 10 | 4 | |
| Wasser, Konservierungsmittel | Ad 100, q.s. | | | | | | | | | | |

### Anhang

| | | | |
|---|---|---|---|
| 1) | Abil^{®} EM 90 | 13) | Cegesoft^{®} C 17 |
| | INCl: Cetyl Dimethicone Copolyol | | INCI: Myristyl Lactate |
| | Hersteller: Tego Cosmetics (Goldschmidt) | | Hersteller. Cognis Deutschland GmbH, Grünau |
| | | | |
| 2) | Allianz^{®} OPT | 14) | Cegesoft^{®} PFO |
| | INCl :Acrylates/C12-22 Alkyl Methacrylate | | INCI: Passiflora Incamata (EU) |
| | Copolymer | | Hersteller: Cognis Deutschland GmbH |
| | Hersteller: Rohm und Haas | | |
| | | | |
| 3) | Amphisol^{®} K | 15) | Cegesoft^{®} PS 6 INCl: Olus |
| | INCI: Potassium Cetyl Phosphate | | Hersteller: Cognis Deutschland GmbH |
| | Hersteller: Hoffmann La Roche | | |
| | | | |
| 4) | Antaron^{®} V 220 | 16) | Ceraphyl^{®}45 INCI: Diethylhexyl Malate |
| | INCl: PVP/Eicosene Copolymer | | Hersteller: International Specialty Products |
| | Hersteller: GAF General Aniline Firm Corp. | | |
| | (IPS-Global) | | |
| | | 17) | Cetiol^{®} 868 |
| 5) | Antaron^{®} V 216 INCI: PVP/Hexadecene Copolymer | | INCI: Ethylhexyl Stearate Hersteller: Cognis Deutschland GmbH |
| | Hersteller: GAF General Aniline Firm Corp. | 18) | Cetiol^{®} A |
| | (IPS-Global) | | INCI: Hexyl Laurate |
| 6) | Ariacel^{®} 83 | | Hersteller: Cognis Deutschland GmbH |
| | INCl: Sorbitan Sesquioleate | 19) | Cetiol^{®} B |
| | Hersteller: Uniqema (ICI Surfacants) | | INCI: Dibutyl Adipate |
| 7) | Arlacel^{®} P 135 | | Hersteller: Cognis Deutschland GmbH |
| | INCl: PEG-30 Dipolyhydroxystearate | 20) | Cetiol^{®} CC |
| | Hersteller : Uniqema (ICI Surfacants) | | INCI: Dicaprylyl Carbonate |
| | | | Hersteller: Cognis Deutschland GmbH |
| | | | |
| 8) | Bentone^{®} 38 INCl: Quatemium-18 Hectorite Hersteller: Rheox (Elementis Specialties) | 21) | Cetiol^{®} J 600 INCI: Oleyl Erucate Hersteller: Cognis Deutschland GmbH |
| | | | |
| 9) | Carbopol^{®} 980 | 22) | Cetiol^{®} LC |
| | INCl: Carbomer Hersteller: Goodrich | | INCI: Coco-Caprylate/Caprate Hersteller: Cognis Deutschland GmbH |
| | | | |
| 10) | Carbopol^{®} 2984 | 23) | Cetiol^{®} OE |
| | INCl: Carbomer Hersteller: Noveon, Inc. | | INCl: Dicaprylyl Ether Hersteller: Cognis Deutschland GmbH |
| | | | |
| 11) | Carbopol^{®} ETD 2001 | 24) | Cetiol^{®} PGL |
| | INCl: Carbomer Hersteller: Noveon, Inc. | | INCI: Hexyldecanol, Hexyldecyl Laurate Hersteller. Cognis Deutschland GmbH |
| | | | |
| 12) | Carbopol^{®} Ultrez 10 | 25) | Cetiol^{®} S |
| | INCl: Carbomer Hersteller: Noveon, Inc. | | INCI: Diethylhexylcyclohexane Hersteller. Cognis Deutschland GmbH |
| 26) | Cetiol^{®} SB 45 | 39) | Dow Corning^{®} 244 Fluid |
| | lNCl: Shea Butter Butyrospermum Parkii | | lNCl: Cyclomethicone |
| | (Linne) | | Hersteller: Dow Corning |
| | Hersteller: Cognis Deutschland GmbH | 40) | Dow Corning^{®} 246 Fluid |
| 27) | Cetiol^{®} SN | | INCl: Cyclopentasiloxane |
| | INCI: Cetearyl Isononanoate | | Hersteller: Dow Corning |
| | Hersteller: Cognis Deutschland GmbH | 41) | Dow Corning^{®} 2502 |
| 28) | Copherol^{®} F 1300 C | | INCI: Cetyl Dimethicone |
| | INCI: Tocopherol | | Hersteller: Dow Corning |
| | Hersteller: Cognis Deutschland GmbH | 42) | Dry^{®}Flo Plus |
| 29) | Copherol 1250 C | | INCl: Aluminium Starch Octenylsuccinate |
| | INCl: Tocopheryl Acetate | | Hersteller: National Starch |
| | Hersteller: Cognis Deutschland GmbH | 43) | Elfacos^{®}ST 37 |
| 30) | Cosmedia^{®} DC | | INCI: PEG-22 Dodecyl Glycol Copolymer |
| | lNCl: Hydrogenated Dimer Dilinoleyl / | | Hersteller: Akzo-Nobel |
| | Dimethylcarbonate Copolymer | | |
| | Hersteller. Cognis Deutschland GmbH | 44) | Elfacos^{®}ST 9 |
| | | | INCI: PEG-45 Dodecyl Glycol Copolymer |
| 31) | Cosmedia^{®} SP | | Hersteller: Akzo-Nobel |
| | INCl: Sodium Polyacrylate | | |
| | Hersteller: Cognis Deutschland GmbH | 45) | Emery^{®} 1780 |
| | | | INCI: Lanolin Alcohol |
| 32) | Cutina^{®} E 24 | | Hersteller: Cognis Corporation (Emery) |
| | INCl: PEG-20 Glyceryl Stearate | | |
| | Hersteller: Cognis Deutschland GmbH | 46) | Emulgade^{®} CM |
| | | | INCl: Cetearyl Isononanoate and Ceteareth-20 |
| 33) | Cutina^{®} HR | | and Cetearyl Alcohol and Glyceryl |
| | INCI: Hydrogenated Castor Oil | | Stearate and Glycerin and Ceteareth-12 |
| | Hersteller: Cognis Deutschland GmbH | | and Cetyl Palmitate |
| 34) | Cutina^{®} MD | | Hersteller: Cognis Deutschland GmbH |
| | INCI: Glyceryl Stearate | 47) | Emulgade^{®}PL 68/50 |
| | Hersteller: Cognis Deutschland GmbH | | INCl: Cetearyl Glucoside, Cetearyl Alcohol |
| 35) | Cuitina^{®} PES | | Hersteller: Cognis Deutschland GmbH |
| | INCI: Pentaerythrityl Distearate | 48) | Emulgade^{®} SE - PF |
| | Hersteller: Cognis Deutschland GmbH | | INCl: Glyceryl Stearate (and) Ceteareth-20 |
| | | | (and) Ceteareth-12 (and) Cetearyl Alcohol |
| 36) | Dehymuls^{®} FCE | | (and) Cetyl Palmitate |
| | INCl: Dicocoyl Pentaerythrityl Distearyl | | Hersteller: Cognis Deutschland GmbH |
| | Citrate | | |
| | Hersteller: Cognis Deutschland GmbH | 49) | Emulgade^{®} SUCRO |
| | | | INCI: Sucrose Polystearate (and) |
| 37) | Dehymuls^{®} HRE 7 | | Hydrogenated Polyisobutene |
| | INCl: PEG-7 Hydrogenated Castor Oil | | Hersteller: Cognis Deutschland GmbH |
| | Hersteller: Cognis Deutschland GmbH | 50) | Eumulgin^{®} B1 |
| 38) | Dehymuls^{®} PGPH | | INCI: Ceteareth-12 |
| | INCI: Polyglyceryl-2 Dipolyhydroxystearate | | Hersteller: Cognis Deutschland GmbH |
| | Hersteller. Cognis Deutschland GmbH | | |
| 51) | Eumulgin^{®} B 2 | | Hersteller: NRC Nordmann/Rassmann |
| | INCI: Ceteareth- 20 | | |
| | Hersteller: Cognis Deutschland GmbH | 64) | Hispagel^{®} 200 |
| | | | INCI: Glycerin, Glyceryl Polyacrylate |
| 52) | Eumulgin^{®} HRE 40 | | Hersteller: Cognis Deutschland GmbH |
| | lNCl : PEG-40 Hydrogenated Castor Oil | | |
| | Hersteller: Cognis Deutschland GmbH | 65) | Hostaphat^{®} KL 340 N |
| | | | INCl: Trilaureth-4 Phosphate |
| 53) | Eumulgin^{®} SG | | Hersteller: Clariant |
| | INCl: Sodium Stearoyl Glutamate | | |
| | Hersteller: Cognis Deutschland GmbH | 66) | Hydagen^{®} C.A.T. |
| | | | INCl Triethyl Citrate |
| 54) | Eumulgin^{®} VL 75 | | Hersteller: Cognis Deutschland GmbH |
| | INCl: Lauryl Glucoside (and) Polyglyceryl-2 | | |
| | Dipolyhydroxystearate (and) Glycerin | 67) | Hydagen^{®} DCMF |
| | Hersteller: Cognis Deutschland GmbH | | INCI : Chitosan |
| | | | Hersteller: Cognis Deutschland GmbH |
| 55) | Eusolex^{®} OCR | 68) | Insect Repellent^{®} 3535 |
| | INCI: Octocrylene | | INCl : Ethyl Butylacetylaminopropionate |
| | Hersteller: Merck | | Hersteller : EMD Chemicals Inc |
| 56) | Eusolex^{®} T 2000 | 69) | Isolan^{®} PDI |
| | lNCl: Titanium Dioxide, Alumina, | | INCI: Diisostearoyl Polyglyceryl-3 Diisostearate |
| | Simethicone | | Hersteller: Goldschmidt AG |
| | Hersteller: Merck | 70) | Keltrol^{®} T |
| 57) | Eusolex^{®} T AQUA | | INCI: Xanthan Gum |
| | INCI: Water and Titanium Dioxide and Alumina | | Hersteller: CP Kelco |
| | and Sodium Metaphosphate and | | |
| | Phenoxyethanol and Sodium | 71) | Lameform^{®} TGI |
| | Methylparaben | | INCI: Polyglyceryl-3 Diisostearate |
| | Hersteller: Merck | | Hersteller: Cognis Deutschland GmbH |
| 58) | Eutanol^{®} G | 72) | Lanette^{®} 14 |
| | INCI: Octyldodecanol | | INCI: Myristyl Alcohol |
| | Hersteller: Cognis Deutschland GmbH | | Hersteller: Cognis Deutschland GmbH |
| 59) | Eutanol^{®}G 16 | 73) | Lanette 18 |
| | lNCl: Hexyldecanol | | INCI: Stearyl Alcohol |
| | Hersteller: Cognis Deutschland GmbH | | Hersteller: Cognis Deutschland GmbH |
| 60) | Eutanol^{®}G 16 S | 74) | Lanette^{®} 22 |
| | lNCl : Hexyldecyl Stearate | | INCl: Behenyl Alcohol |
| | Hersteller: Cognis Deutschland GmbH | | Hersteller: Cognis Deutschland GmbH |
| 61) | Finsolv^{®} TN | 75) | Lanette^{®} E |
| | INCI: C 12/15 Alkyl Benzoate | | INCI: Sodium Cetearyl Sulfate |
| | Hersteller: Findex (NordmannlRassmann) | | Hersteller: Cognis Deutschland GmbH |
| | | 76) | Lanette^{®} O |
| 62) | Generol^{®} R | | INCI: Cetearyl Alcohol |
| | INCl: Brassica Campestris (Rapseed) Sterols | | Hersteller: Cognis Deutschland GmbH |
| | Hersteller: Cognis Deutschland GmbH | 77) | Locron^{®} L |
| 63) | Glucate^{®} DO | | INCI: Aluminium Chlorhydrate |
| | INCI: Methyl Glucose Dioleate | | Hersteller: Clariant |
| 78) | Lucentite^{®} SAN | 83) | Neo Heliopan^{®} OS |
| | INCI: Quatemium-18 Hectoritr | | INCI: Ethylhexyl Salicylate |
| | Hersteller: Co-Op Chemical Co., Ltd. | | Hersteller: Symrise |
| 79) | Monomuls^{®} 90-018 | 84) | Novata^{®} AB |
| | INCI: Glyceryl Oleate | | INCI: Cocoglycerides |
| | Hersteller: Cognis Deutschland GmbH | | Hersteller: Cognis Deutschland GmbH |
| 80) | Myrj^{®} 51 | 85) | Parsol^{®} 1789 |
| | INCI: PEG-30-Sterate | | INCI: Butyl Methoxydibenzoylmethane |
| | Hersteller: Uniqema | | Hersteller: Hoffmann-La Roche (Givaudan) |
| 81) | Myritol^{®} 312 | 86) | Pemulen^{®} TR-2 Polymer |
| | INCl: Caprylic/Capric Triglyceride | | INCl: Acrylates/C10-30Alkylacrylate |
| | Hersteller: Cognis Deutschland GmbH | | Crosspolymer |
| 82) | Myritol^{®} 331 | | Hersteller: Noveon, Inc. |
| | INCI: Cocoglycerides | 87) | Photonyl^{®} LS |
| | Hersteller: Cognis Deutschland GmbH | | INCI: Arginine, Disodium Adenosine |
| | | | Triphosphate, Mannitol, Pyridoxine HCL, |
| 83) | Myritol^{®} PC | | Phenylalanine, Tyrosine |
| | INCI: Propylene Glycol Dicaprylate/Dicaprate | | Hersteller: Laboratoires Serobiologiques |
| | Hersteller: Cognis Deutschland GmbH | | (Cognis) |
| 84) | Neo Heliopan^{®} 303 | 88) | Prisorine^{®} 3505 |
| | INCI: Octocrylene | | INCI: Isostearic Acid |
| | Hersteller: Symrise | | Hersteller: Uniqema |
| 85) | Neo Heliopan^{®} AP | 89) | Prisorine^{®} 3758 |
| | INCI: Disodium Phenyl Dibenzimidazole | | INCl: Hydrogenated Polyisobutene |
| | Tetrasulfonate | | Hersteller: Uniqema |
| | Hersteller: Symrise | 90) | Rezal 36G |
| 86) | Neo Heliopa^{®} AV | | INCI: Aluminum Zirconium Tetrachlorohydrex |
| | INCI: Ethylhexyl Methoxycinnamate | | GLY |
| | Hersteller: Symrise | | Hersteller: Reheis, Inc |
| 87) | Neo Heliopan^{®} BB | 91) | SFE^{®} 839 |
| | INCI: Benzophenone-3 | | INCI: Cyclopentasiloxane and |
| | Hersteller: Symrise | | DimethiconeNinyl Dimethicone Crosspolymer |
| | | | Hersteller: GE Silicones |
| 88) | Neo Heliopan^{®} E 1000 INCI: Isoamyl-p-Methoxycinnamate | 92) | Silikonöl Wacker AK^{®} 350 |
| | Hersteller: Symrise | | INCI: Dimethicone |
| | | | Hersteller: Wacker |
| 81) | Neo Heliopan^{®} Hydro | | |
| | INCI: Phenylbenzimidazole Sulfonic Acid | 93) | Tego^{®} Care 450 |
| | Hersteller: Symrise | | INCI: Polyglyceryl-3 Methylglucose |
| | | | Distearate |
| 82) | Neo Heliopan^{®} MBC | | Hersteller: Tego Cosmetics (Goldschmidt) |
| | INCl: 4-Methylbenzylidene Camphor | | |
| | Hersteller: Symrise | 94) | Tego^{®} Care CG 90 |
| | | | INCl: Cetearyl Glucoside |
| | | | Hersteller: Goldschmidt |
| 95) | Tegosoft^{®} DEC | | |
| | INCI: Diethylhexyl Carbonate | | |
| | Hersteller: Goldschmidt | | |
| 96) | Tinosorb^{®} S | | |
| | INCI: Bis-Ethylhexyloxyphenol Methoxyphenyl | | |
| | Triazine | | |
| | Hersteller. Ciba Specialty Chemicals | | |
| | Corporation | | |
| 97) | Tinosorb^{®} M | | |
| | INCI: Methylene Bis-Benzotriazolyl | | |
| | Tetramethylbutylphenol | | |
| | Herstelller: Ciba Specialty Chemicals | | |
| | Corporation | | |
| 98) | Tween^{®}60 | | |
| | INCl: Polysorbate 60 | | |
| | Hersteller: Uniqema (ICl Surfactants) | | |
| 99) | Uvasorb^{®} HEB | | |
| | INCl: Diethylhexyl Butamido Triazone | | |
| | Hersteller: 3V Inc. | | |
| 100) | Unirep^{®} U-18 | | |
| | INCl: Dimethyl Phthalate and Diethyl Toluamide | | |
| | and Ethyl Hexanediol | | |
| | Hersteller: Induchem AG | | |
| 101) | Uvinul^{®} T 150 | | |
| | INCI: Ethylhexyl Triazone | | |
| | Hersteller: BASF | | |
| 102) | Uvinul^{®} A plus | | |
| | INCI: Diethylamino Hydroxybenzoyl Hexyl | | |
| | Benzoate | | |
| | Hersteller: BASF | | |
| 103) | Veegum^{®} Ultra | | |
| | INCl: Magnesium Aluminium Silicate | | |
| | Hersteller: R. T. Vanderbilt Company, Inc | | |
| 104) | Veegum^{®} Plus | | |
| | INCI: Magnesium Aluminum Silicate and | | |
| | Cellulose Gum | | |
| | Hersteller: R. T. Vanderbilt Company, Inc | | |
| 105) | Z-Cote^{®} HP 1 | | |
| | INCl: Zinc Oxide and Triethoxycaprylylsilane | | |
| | Hersteller: BASF | | |
| 106) | Zinc Oxide NDM | | |
| | INCl: Zinc Oxide | | |
| | Hersteler: Symrise | | |

## Patentansprüche

1. Kohlenwasserstoff Gemisch enthaltend lineare C11 und lineare C13 Kohlenwasserstoffe, wobei die Summe der linearen C11- und linearen C13-Kohlenwasserstoffe größer gleich 60 Gew. % bezogen auf die Summe der Kohlenwasserstoffe, beträgt.

2. Kohlenwasserstoff Gemisch nach Anspruch 1, wobei das Gemisch enthält
a. 50 bis 90 Gew.-% lineare C-11 Kohlenwasserstoffe
b. 10 bis 50 Gew.-% lineare C13 Kohlenwasserstoffe
bezogen auf die Summe der Kohlenwasserstoffe.

3. Kohlenwasserstoff Gemisch nach einem der vorgenannten Ansprüche, **dadurch gekennzeichnet, dass** die Summe der verzweigten Kohlenwasserstoffe kleiner gleich 10 Gew.-%, bezogen auf die Summe der Kohlenwasserstoffe, beträgt.

4. Kohlenwasserstoff Gemisch nach einem der vorgenannten Ansprüche, **dadurch gekennzeichnet, dass** die linearen C11 und/oder linearen C13 Kohlenwasserstoffe gesättigte Kohlenwasserstoffe sind.

5. Kohlenwasserstoff Gemisch nach einem der vorgenannten Ansprüche, wobei der Anteil an **C12 Kohlenwasserstoffe kleiner gleich 10 Gew.-%,** insbesondere kleiner gleich 5 Gew.%, bevorzugt kleiner gleich 3 Gew.-% -bezogen auf die Summe der Kohlenwasserstoffe beträgt.

6. Kohlenwasserstoff Gemisch nach einem der vorgenannten Ansprüche, wobei die Summe der **Kohlenwasserstoffe mit einer C-Kettenlänge größer gleich 14,** kleiner gleich 15 Gew.-%, bezogen auf die Summe der Kohlenwasserstoffe, beträgt.

7. Kohlenwasserstoff Gemisch nach einem der vorgenannten Ansprüche, **dadurch gekennzeichnet, dass** die Summe der **Kohlenwasserstoffe mit einer C-Kettenlänge von kleiner gleich 10,** kleiner gleich 3 Gew.-%, bezogen auf die Summe der Kohlenwasserstoffe, beträgt.

8. Verwendung eines Kohlenwasserstoff Gemisches nach einem der Ansprüche 1 bis 7 in kosmetischen und/oder pharmazeutischen Zubereitungen, insbesondere als Ölkörper.

9. Kosmetische und/oder pharmazeutische Zubereitungen, enthaltend **1 bis 80 Gew.% Kohlenwasserstoffe**, wobei die Summe der linearen C11- und linearen C13-Kohlenwasserstoffe größer gleich 60 Gew. % bezogen auf die Summe der Kohlenwasserstoffe beträgt.

10. Kosmetische und/oder pharmazeutische Zubereitung nach Anspruch 9, enthaltend mindestens **einen Antiperspirant /Desodorant Wirkstoff**.

11. Kosmetische und/oder pharmazeutische Zubereitung nach Anspruch 9, enthaltend mindestens **einen UV-Lichtschutzfilter.**
